# EUROPEAN PATENT SPECIFICATION

(11) **EP 0 628 051 B1**
(45) Date of publication and mention of the grant of the patent: **02.07.2003**
(21) Application number: 93905954.9
(22) Date of filing: 12.02.1993
(51) Int. Cl.: C07H 21/00, C12Q 1/68, C07F 9/6561, C07H 19/04, C07H 19/10, C07H 19/20

(54) **APPLICATIONS OF FLUORESCENT N-NUCLEOSIDES AND FLUORESCENT STRUCTURAL ANALOGS OF N-NUCLEOSIDES**
VERWENDUNGEN VON FLUORESZIERENDEN N-NUKLEOSIDEN UND DESSEN ANALOGEN
UTILISATION DE N-NUCLEOSIDES FLUORESCENTS ET DE LEURS ANALOGUES STRUCTURELS FLUORESCENTS

(30) Priority: 12.02.1992 US 834456
(43) Date of publication of application: 14.12.1994
(73) Proprietor: Chromagen, Inc., San Diego, CA 92121-1503 (US)
(72) Inventor: CONRAD, Michael, J., San Diego, CA 92129 (US)
(74) Representative: Perry, Robert Edward
(86) International application number: PCT/US93/01338
(87) International publication number: WO 93/016094

(56) References cited:
- EP-A- 0 235 301
- US-A- 3 960 840
- PATENT ABSTRACTS OF JAPAN vol. 12, no. 139 (C-491)27 April 1988
- PATENT ABSTRACTS OF JAPAN vol. 11, no. 259 (C-441)21 August 1987
- JOURNAL OF THE AMERICAN CHEMICAL SOCIETY. vol. 103, 1981, GASTON, PA US pages 932 - 933 ROBINS M.J. AND WILSON J.S. 'Smooth and Efficient Deoxygenation of Secondary Alcohols. A General Procedure for the Conversion of Ribonucleosides to 2'-Deoxynucleosides' cited in the application See Scheme 1

### SEQUENCE LISTING

### (1) GENERAL INFORMATION:

(i) APPLICANT: Chromagen, Inc.
(ii) TITLE OF INVENTION: Application of Fluorescent N-Nucleosides and Fluorescent Structural Analogs of N-Nucleosides
(iii) NUMBER OF SEQUENCES: 3
(iv) CORRESPONDENCE ADDRESS:
   (A) ADDRESSEE: David R. Saliwanchik
   (B) STREET: 2421 N.W. 41st Street, Suite A-1
   (C) CITY: Gainesville
   (D) STATE: FL
   (E) COUNTRY: USA
   (F) ZIP: 32606
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER: US
   (B) FILING DATE:
   (C) CLASSIFICATION:
(vii) PRIOR APPLICATION DATA:
   (A) APPLICATION NUMBER: US 07/834,456
   (B) FILING DATE: 12-FEB-1992
   (C) CLASSIFICATION:
(viii) ATTORNEY/AGENT INFORMATION:
   (A) NAME: Saliwanchik, David R.
   (B) REGISTRATION NUMBER: 31,794
   (C) REFERENCE/DOCKET NUMBER: Chrom-1
(ix) TELECOMMUNICATION INFORMATION:
   (A) TELEPHONE: 904-375-8100
   (B) TELEFAX: 904-372-5800

### (2) INFORMATION FOR SEQ ID NO:1:

(i) SEQUENCE CHARACTERISTICS:
   (A) LENGTH: 39 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(iv) ANTI-SENSE: NO
(vi) ORIGINAL SOURCE:
   (A) ORGANISM: Chlamydia trachomatis
   (C) INDIVIDUAL ISOLATE: L2/434/Bu
   (G) CELL TYPE: Bacterium
(vii) IMMEDIATE SOURCE:
   (A) LIBRARY: lambda 1059 recombinant
   (B) CLONE: lamdba gt11/L2/33
(viii) POSITION IN GENOME:
   (A) CHROMOSOME/SEGMENT: omp112 ORF
(xi) SEQUENCE DESCRIPTION: SEQ ID NO:1:
   AACGTTCGAG ACGGACACCC CTTAGGACGA CTTGGTTCG 39

### (2) INFORMATION FOR SEQ ID NO:2:

(i) SEQUENCE CHARACTERISTICS:
   (A) LENGTH: 39 base paire
   (B) TYPE: nucleic acid
   (C) STRANDEDNESS: single
   (D) TOPOLOGY: linear
(ii) MOLECULE TYPE: transcribed DNA or RNA
(iii) HYPOTHETICAL: NO
(iv) ANTI-SENSE: YES
(ix) FEATURE:
   (A) NAME/KEY: Complementary probe
   (C) IDENTIFICATION METHOD: Hybridization to SEQ ID NO. 1
   (D) OTHER INFORMATION: Control for SEQ ID NO. 3
(xi) SEQUENCE DESCRIPTION: SEQ ID NO:2:
   TTGCAAGCTC TGCCTGTGGG GAATCCTGCT GAACCAAGC 39

### (2) INFORMATION FOR SEQ ID NO:3:

(i) SEQUENCE CHARACTERISTICS:
   (A) LENGTH: 39 base pairs
   (B) TYPE: nucleic acid
   (C) STRANDEDNESS: single
   (D) TOPOLOGY: linear
(ii) MOLECULE TYPE: transcribed DNA or RNA
(iii) HYPOTHETICAL: NO
(iv) ANTI-SENSE: YES
(ix) FEATURE:
   (A) NAME/KEY: Analogous complementary probe
   (C) IDENTIFICATION METHOD: Hybridization to SEQ ID NO. 1
   (D) OTHER INFORMATION: Analog to SEQ ID NO. 2
(xi) SEQUENCE DESCRIPTION: SEQ ID NO:3:
   TTGCNNGCTC TGCCTGTGGG GNNTCCTGCT GNNCCNNGC 39

## Description

The present invention relates to the use of fluorescent structural analogues of the non-fluorescent nucleosides commonly found in DNA and RNA.

### Background of the Invention

The six commonly occurring N-nucleosides which predominate in the composition of DNA and RNA from all sources are adenosine, thymidine, uridine, cytidine, guanosine and inosine. These nucleotides do not absorb light at wavelengths >290 nm and are effectively non-fluorescent under physiological conditions.

Nucleotide sequences are commonly utilised in a variety of applications including diagnostic and therapeutic probes which hybridise target DNA and RNA, and amplification of target sequences. It is often necessary, or useful, to label nucleotide sequences.

Hybridisation of specific DNA or RNA sequences typically involves annealing oligonucleotides of from 5 bases to more than 10,000 bases (10 kb). The majority of oligonucleotide probes currently in research use are radioactively labelled; however, because of (a) the short half-lives of the isotopes in common usage, (b) the safety requirements, and (c) the costs of handling and disposal of radioactive probes, convenient and sensitive non-isotopic methods of detection are required for hybridisation diagnostic methods, to achieve widespread acceptance and application.

In general, all non-isotopic methods of detecting hybridisation probes that are currently available depend on some type of derivatisation of the nucleotides to allow for detection, whether through antibody binding or enzymatic processing, or through the fluorescence or chemiluminescence of an attached "reporter" molecule. In most cases, oligonucleotides have been derivatised to incorporate single or multiple molecules of the same reporter group, generally at specific cyclic or exocyclic positions.

At the simplest level, non-nucleoside linkers and labels have been attached to the 3' or 5' end of existing oligonucleotides by enzymatic or chemical methods. Modification of nucleoside residues internal to the sequence of a DNA or RNA strand has proven to be a difficult procedure, since the reaction conditions must be mild enough to leave the RNA or DNA oligomers intact and still yield reaction products which can participate in normal Watson-Crick base pairing and stacking interactions.

Numerous methods for both cyclic and exocyclic derivatisation of the N-nucleoside have been described, including hapten labelling, amino- and thiol-derivatisation, labelling with photobiotin and other biotinylating agents, dU-biotin labelling, 11-digoxigenin-ddUTP labelling, AAIF, bisulfite modification of cytosine, fluorophore derivatisation of DNA probes, direct enzyme labelling, and acridinium ester labelling. Methods for derivatisation of the furanose ring and at the phosphodiester backbone of oligonucleotides have been reported. These include the introduction of internucleotide linkage reporter groups, and of glycosidic reporter groups.

In order to create non-radioactive detectable olignucleotides, it has been necessary to chemically modify the nucleosides typically used in DNA and RNA probes, which has made such probe preparation expensive and laborious; in many cases, the detection chemistries have also proven cumbersome and expensive to use, which has largely been responsible for their failure to find significant application in clinical laboratories. In their applications to hybridisation, other limitations of chemically derivatised probes have also become apparent, as follows:
(1) chemically derivatised dNTPs are generally not cost-effective for use as stock deoxynucleotide triphosphates in PCR amplification. Hence, labelling of amplified DNA is limited to (i) amplification using previously labelled primers, or (ii) annealing with labelled hybridisation probes. Use of the former frequently results in false positives during amplification, owing to non-specific annealing of primers to non-target segments of DNA during amplification, or contamination by amplicons present in the laboratory environment which are residual from previous amplification experiments. Expense and technical difficulties in post-hybridisation processing have largely limited the applications of labelled hybridisation probes to research.
(2) Base pairing is hindered for many oligomers made with derivatised nucleosides through the introduction of bulky or non-hydrogen bonding bases at inappropriate sites in a sequence. Owing to the inherent background chemiluminescence of many clinical samples, even the acridinium ester probes have failed to achieve their theoretical levels of sensitivity. The requirements for post hybridization processing have remained a limitation to such methods.
(3) It has proven difficult to provide non-radioactively labeled probes which may be inexpensively produced in large quantities.
(4) Chemiluminescent probes are short lived and samples so tested are difficult to quantify or to "reprobe" accurately.
(5) Hybridization in most cases is only inferred, is non-quantitative or only semi-quantitative, and is non-automatable.

These limitations have hindered applications of DNA and RNA hybridization probes to clinical laboratory testing and therapeutic uses.

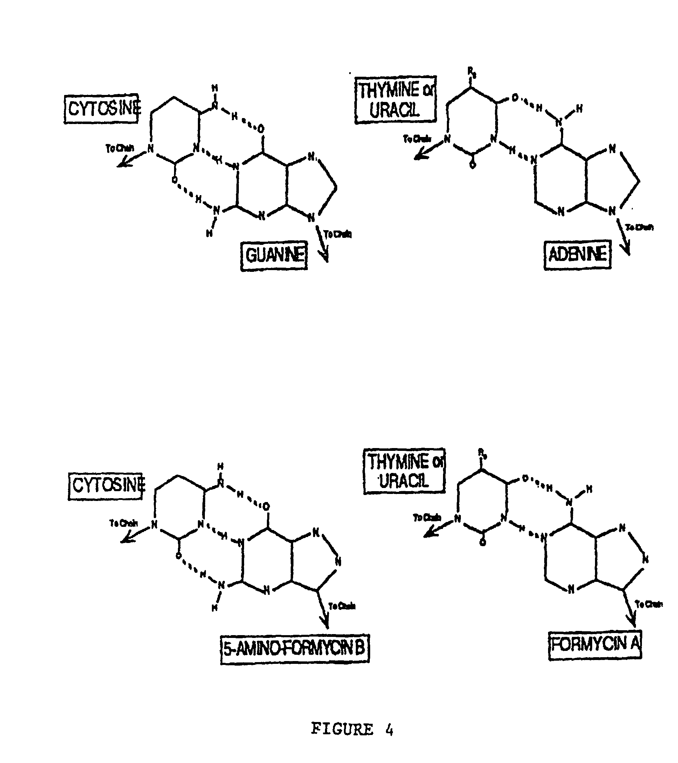

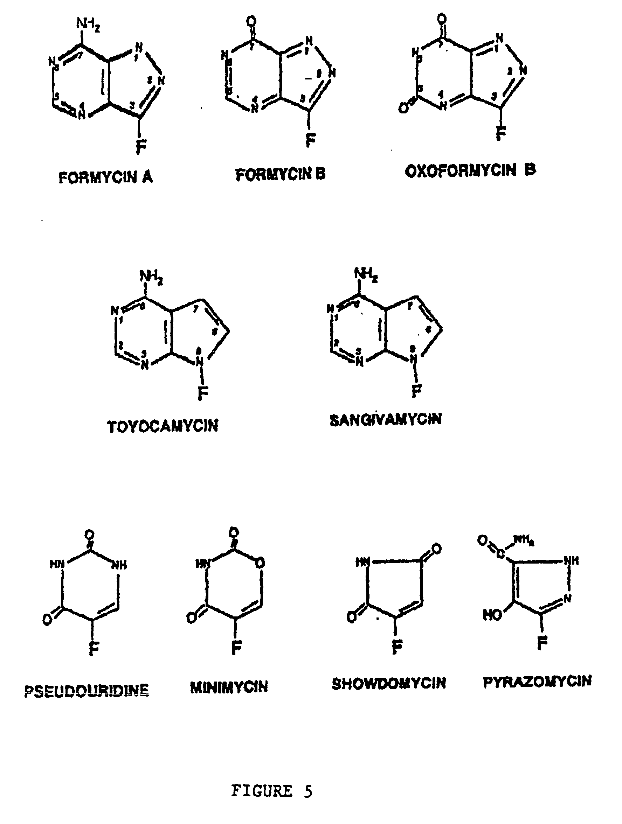

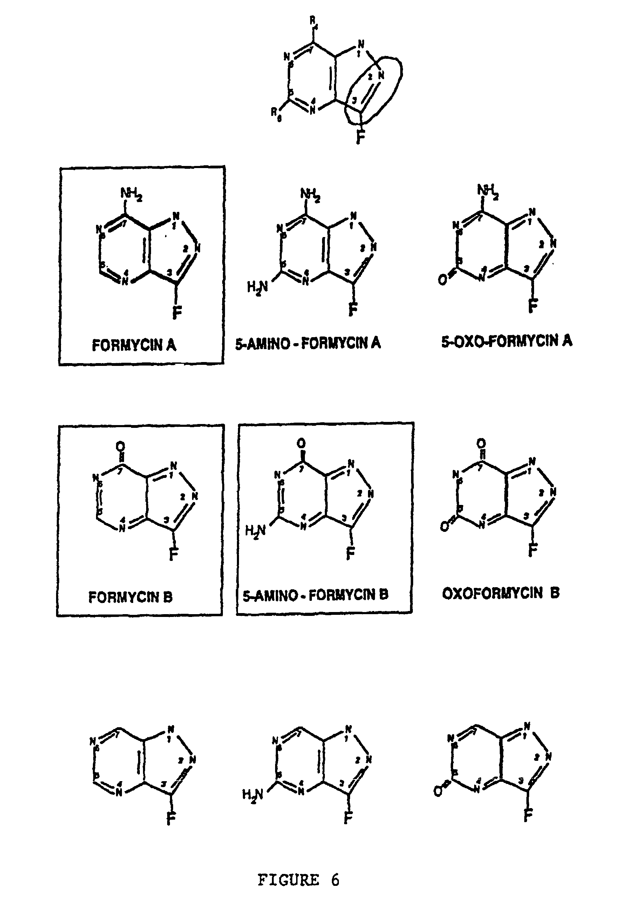

Formycin A (generally referred to as Formycin), the prototypical fluorescent nucleoside analog, was originally isolated as an antitumor antibiotic from the culture filtrates of *Nocardia interforma* (Hori *et al*. [1966] *J. Antibiotics*, Ser. A 17:96-99) and its structure identified as 7-amino-3-b-D-ribafuranosyl (1H-pyrazolo-[4,3d]pyrimidine)) (Figures *5* and *6*). This antibiotic, which has also been isolated from culture broths of *Streptomyces lavendulae* (Aizawa *et al*., [1965] *Agr*. *Biol. Chem*. 29:375-376), and *Streptomyces gummaensis* (Japanese Patent No. 10,928, issued in 1967 to Nippon Kayaku Co., Ltd.), is one of numerous microbial C-ribonucleoside analogs of the N-nucleosides commonly found in RNA from all sources. The other naturally-occurring C-ribonucleosides which have been isolated from microorganisms (Figure 4) include formycin B (Koyama *et al*. [1966] *Tetrahedron Lett*. 597-602; Aizawa *et al*., *supra*; Umezawa *et al*. [1965] *Antibiotics* Ser. A 18:178-181), oxoformycin B (Ishizuka *et al*. [1968] *J. Antibiotics* 21:1-4; sawn *et al*., [1968] *Antibiotics* 21:334-339), pseudouridine (Uematsu and Suahdolnik [1972] *Biochemistry* 11:4669-4674), showdomycin (Darnall *et al*. [1967] *PNAS* 57:548-553), pyrazomycin (Sweeny *et al*. [1973] *Cancer Res*. 33:2619-2623), and minimycin (Kusakabe *et al*. [1972] *J. Antibiotics* 25:44-47). Formycin, formycin B, and oxoformycin B are pyrazolopyrimidine nucleosides and are structural analogs of adenosine, inosine, and hypoxanthine, respectively; a pyrazopyrimidine structural analog of guanosine obtained from natural sources has not been reported in the literature. A thorough review of the biosynthesis of these compounds is available in Ochi *et al*. (1974) *J. Antibiotics* xxiv:909-916.

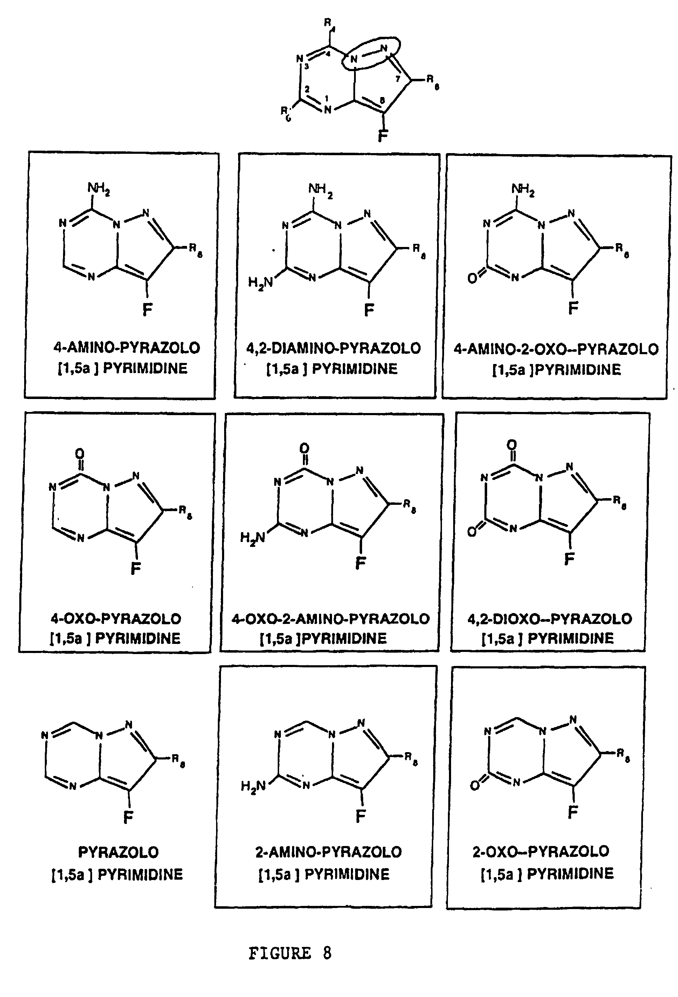

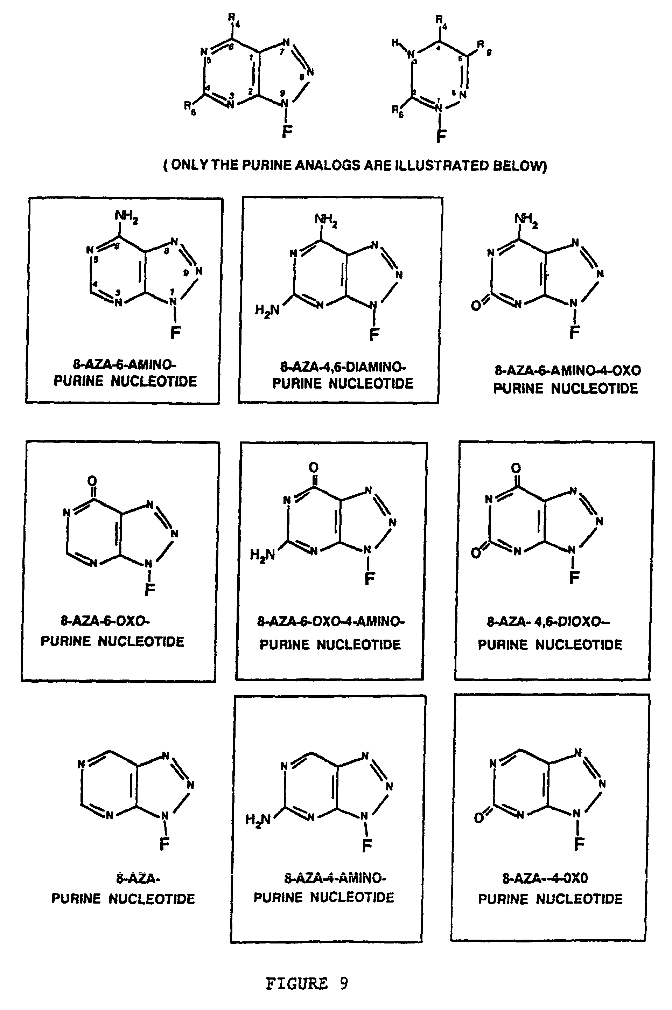

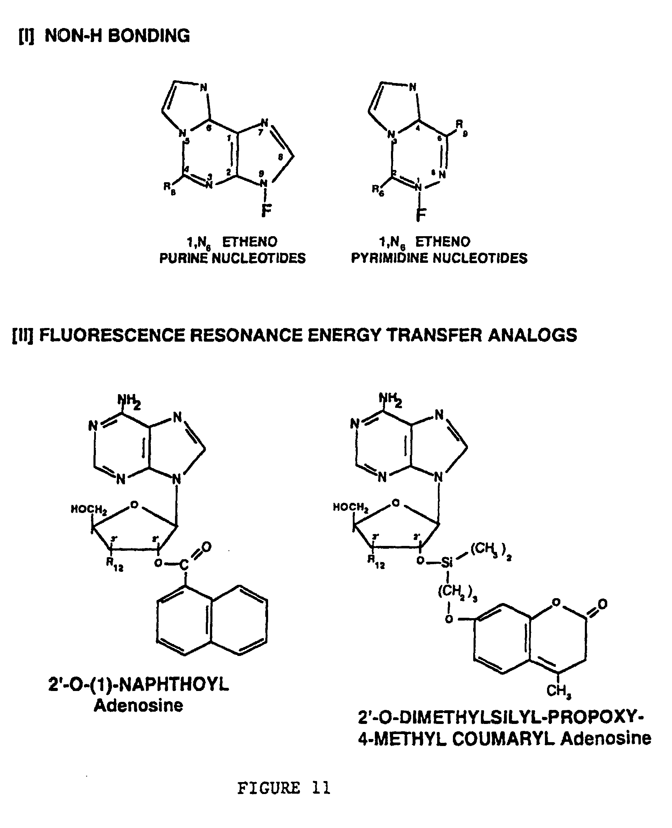

Because several of the C-nucleosides were known to be active as antibiotic, antiviral, or anti-tumor compounds, their chemical derivatization and physical properties have been extensively studied and compared to the structures and syntheses of the N-nucleosides commonly found in DNA and RNA. In the late 1960s, several structural analogs of the six commonly occurring N-nucleosides were found to be fluorescent under physiological conditions; fluorescence in the analogs results from a molecular rigidity of the heterocycle structure itself; not all the structural analogues of a given type, e.g. the C-nucleosides, are fluorescent, nor is fluorescence an exclusive or inherent property of any particular class of structural analogues. Our studies have shown that only a few of the pyrazolo and pyrrolo-pyrimidines and purines are fluorescent, and that the property is shared with a few other nucleoside derivatives and structural analogues including, but not limited to, several substituted N-nucleosides, azanucleosides, ethenonucleosides, and deazanucleosides, the structures of which are shown in Figures 5-11. Those structures in Figures 5-11 which are shown surrounded by boxes have been either previously reported or found to be fluorescent during development of the present invention.

Uncharacterised oligomers containing fluorescent analogues were prepared by Ward and colleagues for physical studies using then available nucleoside polymerase enzymes (Ward *et al*, [1969], J. Biol. Chem. 244:3243-3250; Ward *et al*, [1969] *loc cit* 1228-1237).

EP-A-0235301 and US-A-3960840 disclose the fluorescent nucleoside 1,N⁶-ethenoadenosine and its use as a fluorescent probe.

### Summary of the Invention

According to the present invention, a method for the detection of a target nucleotide in a sample, comprises contacting the sample, under hybridisation conditions, with an oligonucleotide probe that specifically hybridises with the target, and detecting hybridisation by observing fluorescence or a change in fluorescence; characterised in that the probe includes a fluorescent nucleoside instead of any of the six commonly occurring non-fluorescent nucleosides.

In use of the present invention, the fluorescence of the oligonucleotide probe can be used as a diagnostic tool to detect and identify specific genetic sequences. This methodology is distinct from other non-radioactive methods of probe detection in that it does not utilise nucleotides which have been coupled to enzymes or other reactive proteins and does not require post-hybridisation processing for the detection of hybridisation.

For use in the invention, fluorescent structural analogues of the commonly occurring nucleosides and their derivatives useful in the synthesis, labelling, and detection of oligonucleotides, have the structural formulae of Figures 5-11. The commonly occurring nucleosides characteristically form hydrogen bonds in a specific donor/acceptor relationship, designated Watson-Crick base pairing, as shown in Figure 4. Where appropriate, specific fluorescent nucleoside analogues capable of reproducing the pattern of Watson-Crick hydrogen bond formation analogous to that of a particular commonly occurring nucleoside are provided, e.g. as indicated for A:T and formycin:T in Figure 4 by the donor/acceptor patterns.

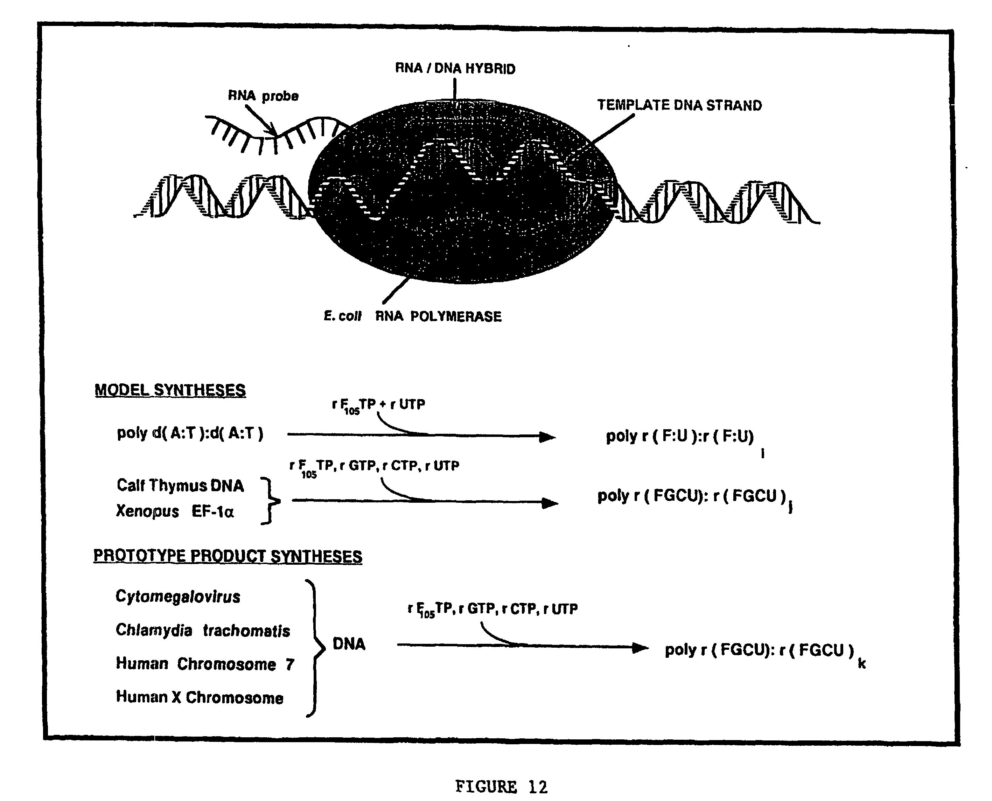

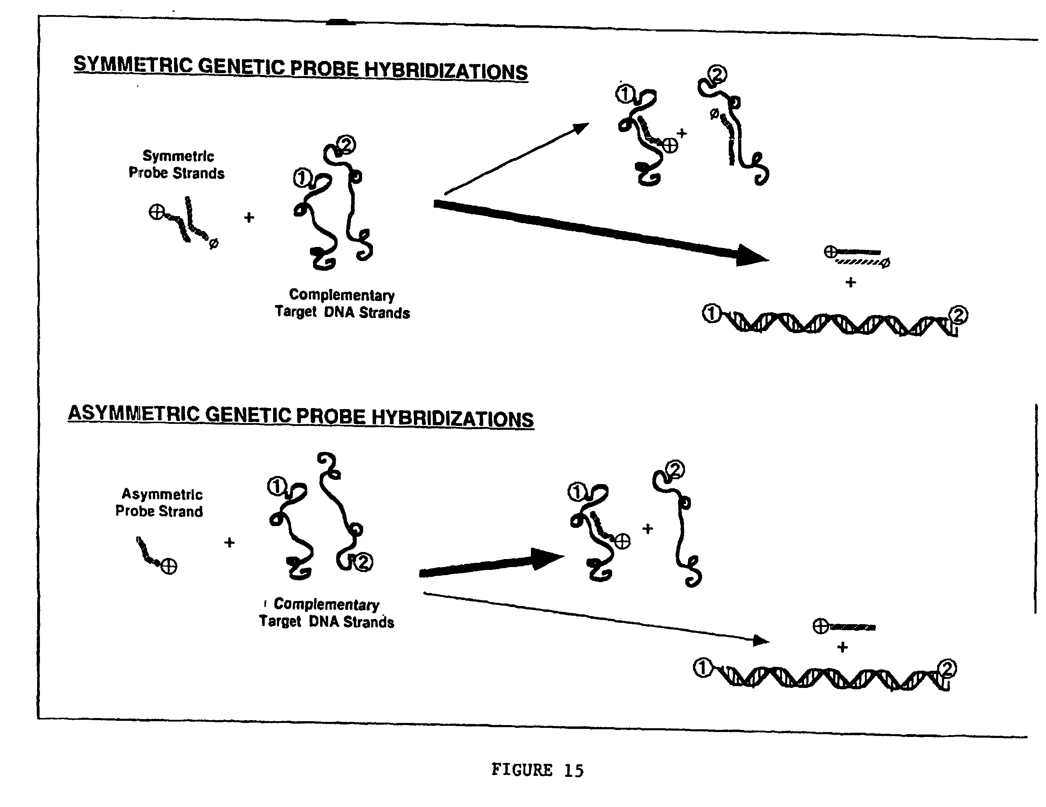

In still another aspect, methods of synthesizing and using polynucleotide probes are provided using one or more of the fluorescent structural analogs and/or their derivatized forms. Such probes can be used to screen a sample containing a plurality of single stranded or double stranded polynucleotide chains and will label, detect, and identify the desired sequence, if present, by hybridization. It is an important aspect of the invention that the fluorescent oligonucleotide probes can be used with "solution hybridization" methods as depicted in Figures 12 through 18.

In accordance with the foregoing objects, the present invention comprises inherently fluorescent nucleosides which can be used to label, modify, or identify oligonucleotides made therefrom, the uses of such inherently fluorescent oligonucleotides as hybridization probes, and methods for detecting nucleotide sequences.

An important aspect of the invention is the stable fluorescence emission of the fluorophores and the use of time-resolved spectroscopy or photon counting to detect and to quantify the amount of a fluorophore present in a sample.

Additional formulae, advantages, methods of use, and novel features of the invention will be set forth in the description which follows, and in part become apparent to those skilled in the art after examination of the following, or may be learned by practice of the invention.

### Brief Description of the Drawings

**Figure 1** shows the six commonly-occurring N-nucleosides which predominate in DNA and RNA.
**Figure 2** shows the general structures of the commonly-occurring N-nucleosides and their derivatization sites, Rₙ.
**Figure 3** shows the general structure of the furanose ring of both the purine and pyrimidine nucleosides and the common sites, Rₙ for derivatization.
**Figure 4** shows Watson-Crick base pairing between the normally occurring N-nucleotides A:T and G:C and base pairing between formycin:T, formycin:U, 2,6-diaminopurine:T, and 5-amino-formycin B:C.
**Figure 5** shows structural analogs of the commonly-occurring N-nucleosides derived from biological sources.
**Figure 6** shows the pyrazolo [4,3d] pyrimidine nucleoside analogs.
**Figure 7** shows the pyrazolo [3,4d] pyrimidine nucleoside analogs.
**Figure 8** shows the pyrazolo [1,5a]-1,3,5-triazine nucleoside analogs.
**Figure 9** shows the azapyrimidine and azapurine nucleoside analogs.
**Figure 10** shows the deazapyrimidine and deazapurine nucleoside analogs.
**Figure 11** shows examples of some fluorescent structural analogs which are (I) non-H-binding, and (II) fluorescence resonance energy transfer (FRET) analogs.
**Figure 12** is a diagram of symmetric RNA synthesis using FTP or ATP.
**Figure 13** is a diagram of promoter directed asymmetric RNA probe synthesis using viral promoters and viral RNA polymerases.
**Figure 14** is a diagram showing an example of the method for one-step labeling of ssDNA inserted at the *Eco*RI site of pUC/M13 plasmid vectors and using dF₁₀₅.
**Figure 15** is a diagram showing the necessity of using asymmetric DNA or RNA probes for rapid and quantitative hybridization of the probe to target DNA. As shown, asymmetric probes provide significant increases in hybridization efficiencies when compared with symmetric probes.
**Figure 16** is a diagram showing the conversion of the ribonucloetide analog, formycin A, to its 2'-deoxy triphosphate or phosphoramidite forms.
**Figure 17** is a diagram of detection of a target DNA sequence in genomic DNA hybridization with fluorescent probes.
**Figure 18** is a diagram of detection of an amplified DNA segment by solution hybridization of a fluorescent probe.
**Figure 19** shows a flow chart diagramming the separation scheme used to separate reaction products from unreacted reagents following the enzymatic substitution reaction of FTP for ATP in RNA probes.
**Figure 20** shows a schematic of the mechanism for increasing detection sensitivity by the use of a probe "cocktail" which contains multiple probes of different sequences.
**Figures 21A, 21B, and 21C** show specific fluorescent nucleoside analogs which have been identified and characterized as to their class, structure, chemical name, absorbance spectra, emission spectra, and methods of synthesis.

### Brief Description of the Sequences

**SEQ ID NO. 1** is a synthetic oligonucleotide according to the subject invention.

**SEQ ID NO. 2** is a synthetic oligonucleotide and the complement of SEQ ID NO. 1.

**SEQ ID NO. 3** is a synthetic oligonucleotide and a fluorescent analog of SEQ ID NO. 2.

### Detailed Disclosure of the Invention

Disclosed and claimed are methods of use of the fluorescent nucleosides in, for example, nucleic acid probes and diagnostic kits. One preferred embodiment pertains to the use of inherently fluorescent nucleoside analogs in the chemical and enzymatic synthesis of DNA hybridization probes including solid phase synthesis, template directed enzymatic polymerization and amplification using polymerase chain reaction methods. Another embodiment relates to the use of autofluorescent DNA hybridization probes in the identification of specific DNA sequences, e.g., gene mapping and the detection and diagnosis of infectious and genetic diseases.

Disclosed are nucleoside analogs which are fluorescent and which can be substituted for naturally occurring nucleosides in the synthesis of oligonucleotide probes. When used as hybridization probes, the fluorescence of such oligonucleotides can be used in a variety of procedures to detect and identify specific genetic sequences. This methodology is distinct from other non-radioactive methods of probe detection in that it does not utilize nucleotides which have been coupled to enzymes or other reactive proteins. Thus, described herein are applications of inherently fluorescent nucleoside analogs in developing hybridization techniques for routine, automatable clinical diagnosis.

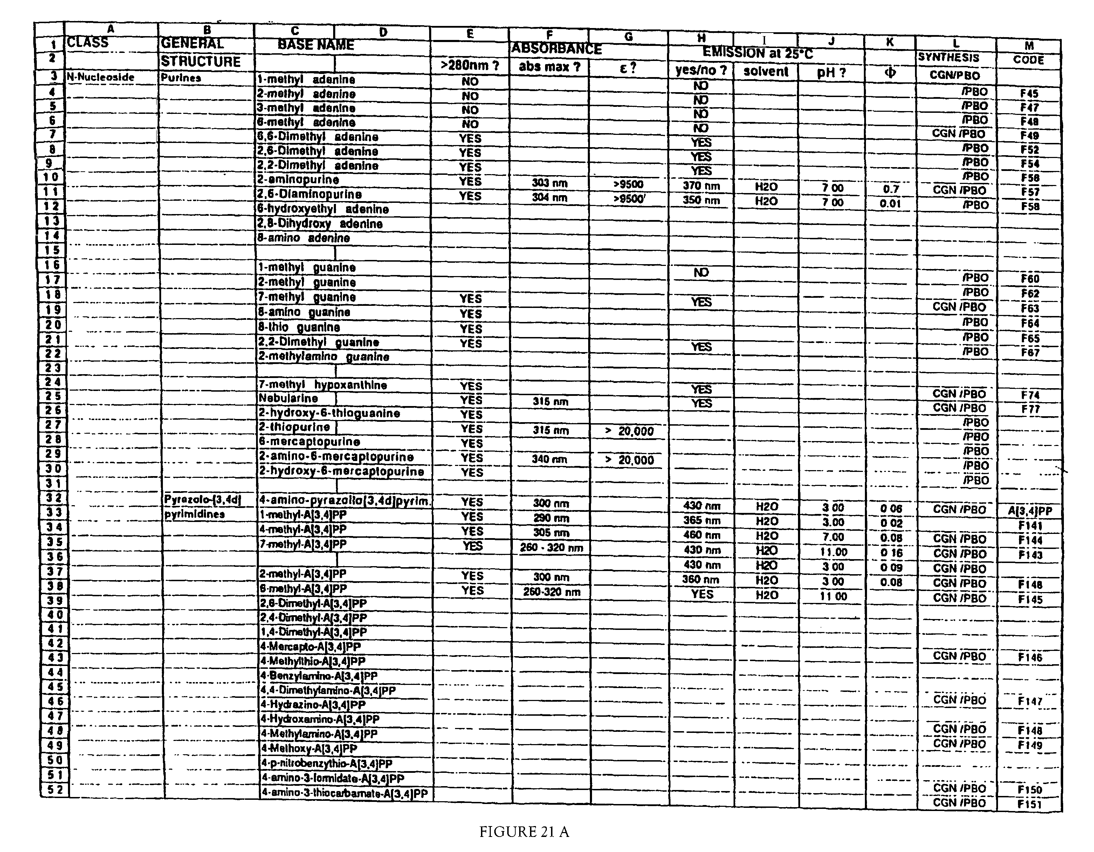

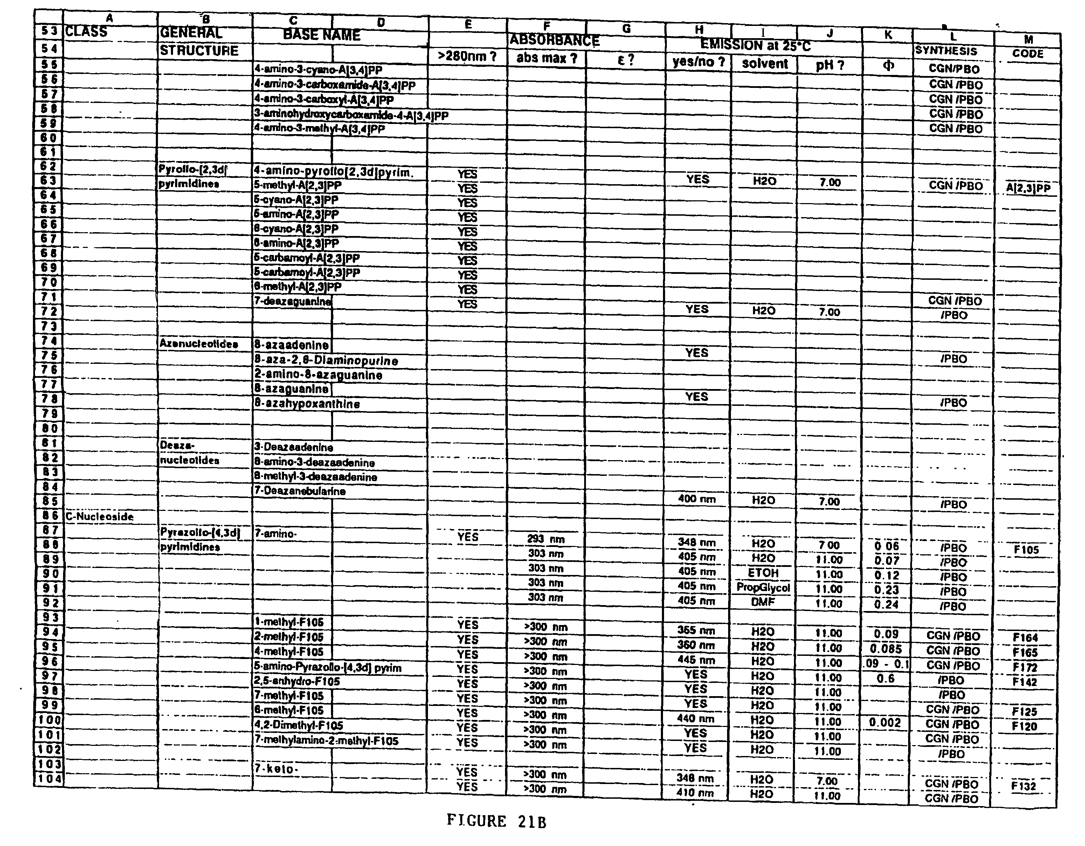

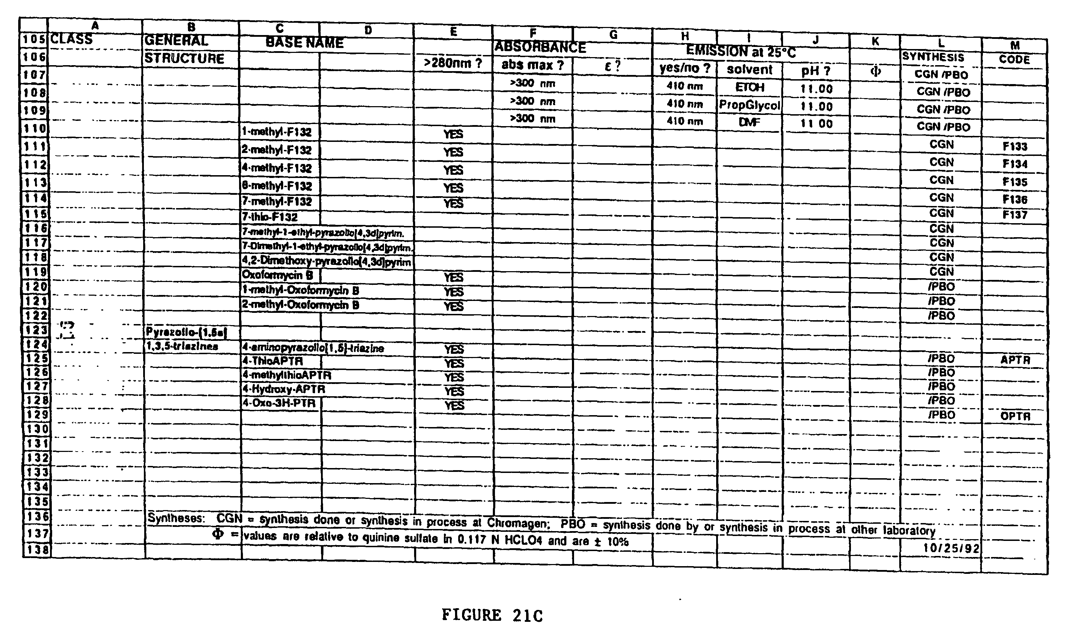

The fluorescent analogs are of three general types: (A) C-nucleoside analogs; (B) N-nucleoside analogs; and (C) N-azanucleotide and N-deazanucleotide analogs. All of these compounds have three features in common: 1) they are structural analogs of the common nucleosides capable of replacing naturally occurring nucleosides in enzymatic or chemical synthesis of oligonucleotides; 2) they are naturally fluorescent when excited by light of the appropriate wavelength(s) and do not require additional chemical or enzymatic processes for their detection; and 3) they are spectrally distinct from the nucleosides commonly encountered in naturally occurring DNA. At least 125 specific compounds of the subject invention have been identified. These compounds, which have been characterized according to their class, structure, chemical name, absorbance spectra, emission spectra, and method of synthesis, are tabulated as shown in Figures 21A-21C.

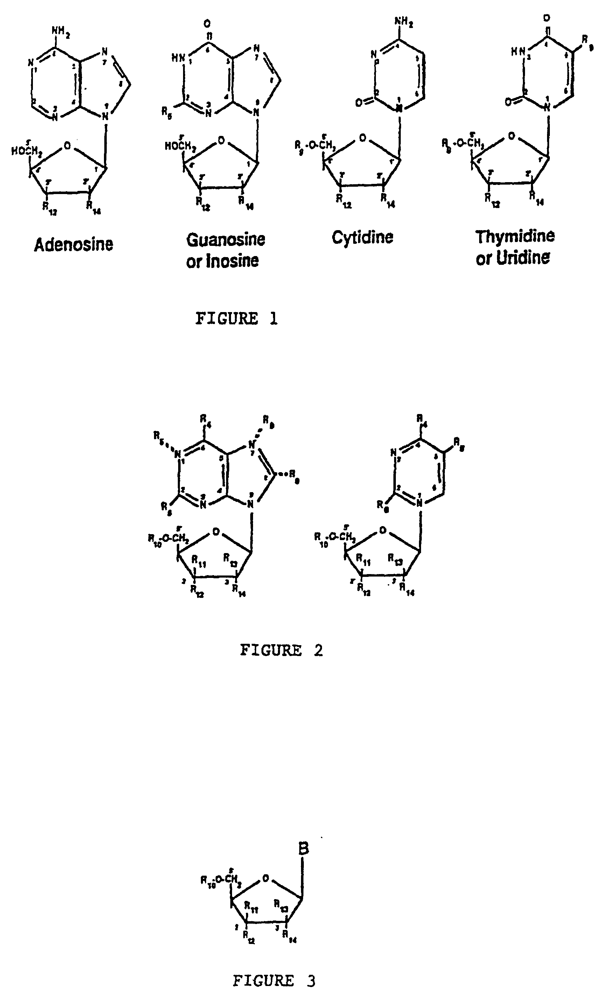

Definitions. The following definitions are provided for ease in understanding the description:
"Commonly Occurring Nucleosides" are the six monomeric N-nucleotides shown in Figure 1, which predominate in naturally occurring DNA and RNA, enter into classical Watson-Crick base pairing, and are effectively non-fluorescent under physiological conditions. The respective one-letter symbols in sequence shorthand are A, C, G, T, U, and I for adenosine, cytidine, guanidine, thymidine, uridine, and inosine, respectively.
"Structural Analogs" of the commonly occurring nucleosides are structurally related molecules that mimic the normal purine or pyrimidine bases in that their structures (the kinds of atoms and their arrangement) are similar to the commonly occurring bases, but may have certain modifications or substitutions which do not affect basic biological activity or biochemical functions. Such base analogs include, but are not limited to, imidazole and its 2,4- and/or 5-substituted derivatives; indole and its 2-, 3-, 4-, 5-, 6-, and/or 7-substituted derivatives; benzimidazole and its 3-, 4-, and/or 5-substituted derivatives; indazole and its 3-, 4-, 5-, 6-, and/or 7- substituted derivatives; pyrazole and its 3-, 4-, and/or 5-substituted derivatives; triazole and its 4- and/or 5-substituted derivatives; tetrazole and its 5-substituted derivatives; benzotriazole and its 4-, 5-, 6-, and/or 7-substituted derivatives; 8-azaadenine and its substituted derivatives; 6-azathymine and its substituted derivatives; 6-azauracil and its substituted derivatives; 5-azacytosine and its substituted derivatives; 8-azahypoxanthine and its substituted derivatives; pyrazolopyrimidine and its substituted derivatives; 3-deazauracil; orotic acid; 2,6-dioxo-1,2,3,6-tetrahydro-4-pyrimidine carboxylic acid; barbituric acid; uric acid; ethenoadenosine; ethenocytidine; an allopurinol (4-hydroxy-pyrazolo [3,4d] pyrimidine); or their protected derivatives as described below. Base analogs can also be any of the C-nucleosides such as are shown in Figures 4 and 5 in which the normal C-N bond between the base and the furanose ring is replaced by a C-C bond; such bases include, but are not limited to, uracil, as in the C-nucleoside pseudouridine; 1-methyluracil;1,3-dimethyluracil;5(4)-carbomethoxy-1,2,3-triazole;5(4)-carboxamido-1,2,3-triazole; 3(5)-carboxymethylpyrazole; 3(5)-carbomethoxypyrazole; 5-carboethoxy-1-methylpyrazole; maleimide (in the C-nucleoside showdomycin); and 3(4)-carboxamido-4(3)-hydroxypyrazole (in the C-nucleoside pyrazomycin); and any of the other analogs listed or inferred in Figures 5 through 11; or their protected derivatives.
"Fluorophore" refers to a substance or portion thereof which is capable of emitting fluorescence in a detectable range. For the fluorescent structural analogs of the nucleotides, this fluorescence typically occurs at wavelengths in the near ultraviolet (>300 nm) through the visible wavelengths. Preferably, fluorescence will occur at wavelengths between 300 nm and 700 nm and most preferably in the visible wavelengths between 300 nm and 500 nm.
"Fluorescent Structural Analogs" are synthetic or biochemically derived monomeric structural analogs of the six commonly occurring N-nucleosides (Figure 1), such as are depicted in Figures 5 through 11, which may or may not be capable of classical Watson-Crick base pairing depending upon the monomeric structure and/or oligonucleotide in which they are used, but which are spectrally unique and distinct from the commonly occurring nucleosides in their capacities for selective excitation and emission under physiological conditions. For example, the C-nucleoside formycin A is a structural analog of adenosine that can form equivalent donor/acceptor hydrogen bonds, but which has an excitation maximum in oligonucleotides at 303 nm and an emission maximum at 405 nm (Stokes Shift = 102 nm).
"Derivatized" nucleoside analogs are fluorescent structural analogs in which reactive or protective functional groups are bound, covalently or otherwise, at the R₄ through R₉ positions of the heterocycle and/or the R₁₀(5'), the R₁₂(3'), and R₁₄(2') positions of the glycosidic moiety. Derivatives at the 2' glycosidic position may include fluorescence resonance energy transfer (FRET) acceptors or donors which enhance or accept and re-emit at longer wavelengths the inherent fluorescence emission of the fluorescent structural analog itself.
A "polynucleotide," "oligonucleotide," or "oligomer" is a nucleotide chain structure containing at least two commonly occurring nucleotides or fluorescent structural analogs. The "fluorescent oligonucleotide probe" or "fluorescent hybridization probe" provided herein is a nucleotide chain structure, as above, containing at least two monomers, at least one of which is fluorescent.
"Hybridization" is the pairwise annealing through Watson-Crick base pairing of two complementary, single-stranded molecules (see Figure 4), which may be DNA:DNA, DNA:RNA, or RNA:RNA, and in which the two strands may come from different sources. The annealing is specific (i) for complementary base pairs in which the hydrogen bond donors and acceptors are oriented as in Figure 4, and (ii) for the complementary genetic sequence of the specific gene, target DNA, or target RNA (hereinafter "target DNA/RNA") to which the probe is to be hybridized. Compare, for example, the hydrogen bond pattern of adenosine and formycin (Figure 4).
"DNA/RNA Melting Temperature" and "Tm" refer to the temperature at which the hydrogen bonds between hybridized strands of DNA or RNA are disrupted and the strands disassociate into single strands, thereby disrupting the structure of the duplex or hybrid.
"Analogous fluorescent sequence" refers to the nucleoside sequence of a polynucleotide which has been synthesized by any of the enzymatic or chemical methods described in the present invention, but in which fluorescent nucleoside analogs have been explicitly substituted for particular commonly occurring nucleosides, e.g., the substitution of formycin A-5'-triphosphate (FTP) for adenosine-5'-triphosphate (ATP), when using RNA polymerase to produce RNA probes complementary to a prescribed DNA template. In an analogous fluorescent sequence, the fluorescent nucleoside analog has been substituted in the oligonucleotide chain at some or all positions in which the corresponding commonly occurring nucleotide would have occurred in the sequence as dictated by, e.g., the template, in the case of enzymatic synthesis. Similar programmed substitutions can be made using 3'-O-phosphoramidites of the individual fluorescent analogs during standard phosphotriester synthesis. Thus, for example, the complementary sequence of the Chlamydia tracheomatis MOMP gene. or its fluorescent analogous sequence, can be synthesized enzymatically using dATP or dFTP, respectively, in the presence of DNA polymerase, dCTP, dTTP, and dGTP:

wherein the fluorescent deoxyformycin A (F) residues underlined in the analogous sequence are the structural analogs of the deoxyadenosine (A) residues in the same relative positions in the complementary sequence.

"FRET acceptor" or "Fluorescence Resonance Energy Transfer acceptor" refers to a substance, substituent, chromophore, or fluorophore, e.g., a dansyl, naphthyl, anthryl, pyrenyl, methylumbelliferone, or coumarin moiety, which is capable of absorbing emitted light from fluorescent structural analog donors and re-emitting that energy at other, longer wavelengths. In the context of the present invention, such secondary fluorophores may be selectively excited as a second label, or may be used as a fluorescence acceptor to broaden and enhance the primary fluorescence of the structural analog energy donor.

### A. Structures, Sources, Synthesis, and Derivatization of the Fluorescent Nucleoside Analogs

Briefly, the present invention includes the heterocyclic pyrimidine or purine structural analogs of the commonly occurring nucleoside bases (B) which are fluorescent under physiological conditions and which are linked by a carbon-carbon or carbon-nitrogen bond to the set of furanose rings (designated F in Figures 4-9) of ribose (R₁₂=R₁₄=OH), deoxyribose (R₁₂=H, R₁₄=OH, or R₁₂=OH, R₁₄=H), or dideoxyribose (R₁₂=R₁₄=H) and their derivatives such as are described below, and/or are apparent to one familiar with nucleotide chemistry.

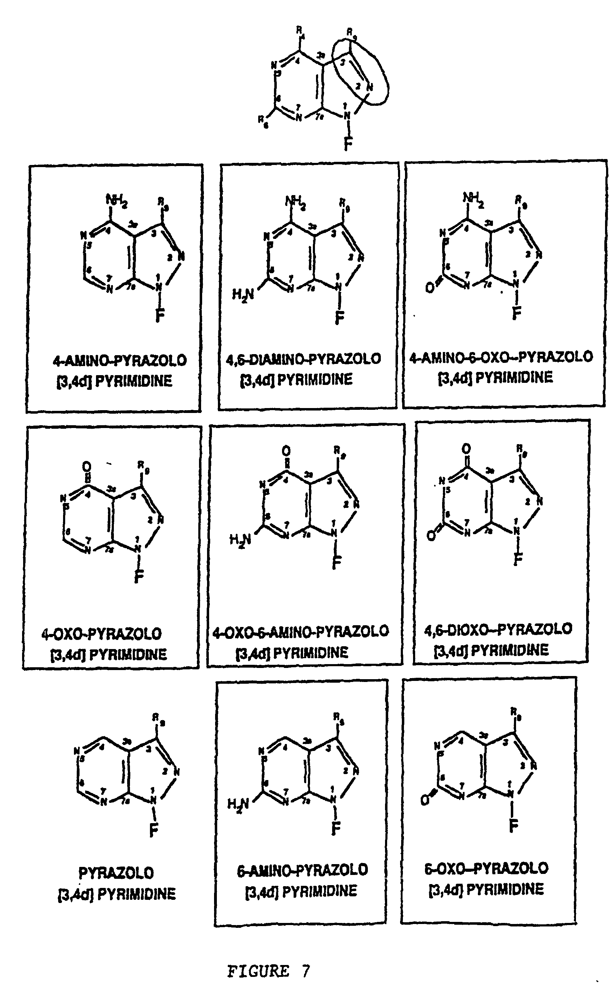

For the present invention, formycin, 2-amino purine ribonucleoside, and 2,6-diamino ribonucleoside, all of which can (i) form the same or related base-pairing hydrogen bonds as adenosine, and (ii) substitute specifically for adenosine in Watson-Crick base pairing as well as in a wide variety of enzymatic reactions including nucleic acid replication, ligation, and phosphorylation, are used as representatives of the set of fluorescent nucleosides and nucleoside analogs (Figure 4). Related properties and parallel claims obtain in the present invention for all other fluorescent analogs of guanosine, cytidine, thymidine, uridine, inosine, and their derivatives.
1. Structures of the nucleoside analogs. The generic purine and pyrimidine structures of each type of structural analog to the commonly occurring nucleosides are given at the top of each of Figures 5 through 11, below which are representative examples of each class of analog. Only examples of the purine analogs are given in Figures 6 and 7, since the known pyrimidine analogs have already been illustrated in Figure 5. With the exception of the N-nucleoside analogs, which have only substitutions at R₄, R₆, and R₉, the generic structures at the top of each page show an oval encircling the part of the structure where substitutions to the heterocyclic base distinguish the analog from the commonly occurring N-nucleosides shown in Figure 1.
2. Furanose moieties common to the fluorescent nucleoside analogs. The numbering of the sugar carbon atoms in furanose is 1' to 5' is indicated in Figure 2, thus the base, B, is connected to C1 of the sugar. The furanose moiety of any fluorescent heterocycle claimed in this invention has, in common with all other analogs, the set F, of glycosides and substituted glycosides, as follows: substitutions can be made, in principle, at any of the 5 sugar carbons; the subset Fis defined by derivatives and/or substitutions at positions R₁₀, R₁₁, R₁₂, R₁₃, and R₁₄, which (i) are obvious to one skilled in the art, and (ii) are the furanosyl derivatives of all the fluorescent nucleoside analogs claimed in the present invention. These include all phosphorous substitutions (e.g., triphosphate, thiophosphate, aminophosphate, etc.) and all protecting substitutions (e.g., dimethoxytrityl) at position R₁₀. For all glycosides, F, in Figures 5 through 11, R₁₀, R₁₁, R₁₂, R₁₃, and R₁₄ are defined as follows: R₁₁ and R₁₃ = H; R₁₄ = H, OH, or ORᵢ; R₁₂ and R₁₀ are either H, OH, ORₘ, or NHRₖ, wherein (a) Rᵢ protecting groups are typically lower aryl or alkyl ether, eg., methyl, t-butyl, benzyl, o-nitrobenzyl, p-nitrobenzyl, o-nitrophenyl, or triphenylmethyl; or a lower alkyl or aryl ester such as acetyl, benzoyl, or p-nitrobenzoyl, or an alkyl; acetal such as tetrahydropyranyl; or a silyl ether, such as trimethylsilyl or t-butyl-dimethylsilyl; or a sulfonic acid ester such as p-toluenesulfonyl or methanesulfonyl; or halide such as bromine, fluorine, or iodine. Additional examples of suitable blocking groups may be found in Green, T.W. (1981) *Protective Groups in Organic Synthesis*, New York: Wiley & Sons. Alternatively, R₁₄ may be a FRET derivative including, but not limited to, such fluorophores as 7-[3-(chlorodimethylsilyl)propoxy]-4-methylcoumarin, O-4-methylcoumarinyl-N-[3-triethoxysilyl)propylcarbamate, and N-3-triethoxysilylpropyl)dansylamide; (b) Rₘ represents an appropriate protecting, substituting, or reactive linker group including 2' or 3'-amido, 2' or 3'-azido, 2',3'-unsaturated, and the subset of phosphorous derivatives involved in chemical or enzymatic syntheses of oligonucleotides having a phosphate ester, thiophosphate ester, or aminophosphate ester backbone; (c) Rₖ is any common, standard nitrogen protecting group, such as those commonly used in peptide synthesis (Geiger, R., W. Konig [1981] In *The Peptides: Analysis, Synthesis, Biology*, VoL 3, E. Gross, J. Meienhofer, eds., Academic Press, New York, pp. 1-99); this includes, but is not limited to, add-labile protecting groups such as formyl, t-butyloxycarbonyl, benzyloxycarbonyl, 2-chlorobenzyloxycarbonyl, 4-chlorobenzyloxycarbonyl, 2,4-dichlorobenzyloxycarbonyl, furfuryloxycarnonyl, t-amyloxycarbonyl, adamantyloxycarbonyl, 2-phenylpropyl-(2)-oxycarbonyl, 2-(4-biphenyl)propyl-(2)-oxycarbonyl, triphenylmethyl, p-anisyldiphenylmethyl, di-p-anisyl diphenylmethyl, 2-nitrophenylsulfenyl, or diphenylphosphinyl; base labile protecting groups such as trifluoroacetyl, 9-fluorenylmethyloxycarbonyl, 4-toluene-sulfonylethyloxycarbonyl, methylsulfonylethyloxycarbonyl, and 2-cyano-t-butyloxycarbonyl; as well as others, such as chloroacetyl, acetoacetyl, 2-nitro-benzoyl, dithiasuccinoyl, maleoyl, isonicotinyl, 2-bromoethyloxycarbonyl, and 2,2,2-trichloroethyloxycarbonyl; alternatively, Rₖ may also be any reactive group derivatizible with a detectable label (NH₂, SH, =O, and which can include an optional linking moiety including an amide, thioether or disulfide linkage, or a combination thereof with additional variable reactive groups R₁ through R₃, such as R₁-(CH₂)ₓ-R₂, where x is an integer in the range of 1 and 8, inclusive; and R₁, R₂, and R₃ are H, OH, alkyl, acyl, amide, thioether, or disulfide) or any linker or spacer functioning as a homobifunctional or heterobifunctional linker including, but not limited to, such reactive groups as hydrazides, maleimidazoles, oxidizable diols, and succinimydyl groups. At most only one of R₁₂ and R₁₀ may be NHRₖ.

Reference is made to phosphoramidites having the formula: 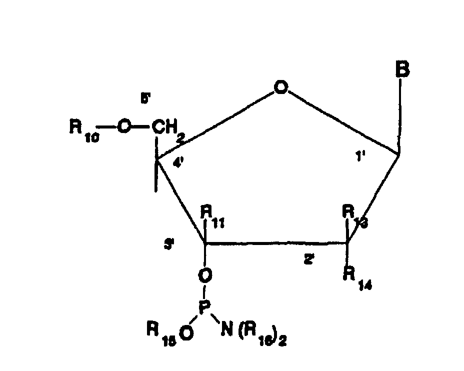 wherein B is any of the fluorescent nucleoside analogs described herein and R₁₀, R₁₁, R₁₂, R₁₃ are as defined for the set of glycosides, F, as above, and R₁₄ may be either H or OH. R₁₆ = lower alkyl, preferably lower alkyl such as methyl or isopropyl, or heterocyclic, such as morpholino, pyrrolidono, or 2,2,6,6-tetramethylpyrrolidono; R₁₅ = methyl, beta-cyanoethyl, p-nitrophenyl, o-chloronitrophenyl, or p-chlorophenyl. All other R groups are as before including those identifying spacer or linker arms of from 1 to 25 carbon atoms in length. Prior to the synthesis of the phosphoramidite at R₁₂ in order to (i) preserve any reactive substituents on the heterocycle which are important to its participation in Watson-Crick base pairing, and (ii) render the amidite compatible with the DNA or RNA chain assembly chemistry, the base moiety B in the phosphoramidite can be protected, which generally involves acylation or amidation of the exocyclic amino groups and includes, but is not limited to, acetyl, benzoyl, isobutryl, succcinyl, phthaloyl, or p-anisoyl; such amidine groups include, but are not limited to, dimethylformamidme, di-n-butylformamidine, or dimethylacetamidine; if B is substituted with other reactive groups such as carboxyl, hydroxyl, or mercapto, these are appropriately protected as well.

The present invention encompasses the synthesis of oligonucleotides on a solid phase support, wherein the oligomer is reacted with the protected fluorescent nucleoside analog phosphoramidites as illustrated in Figures 5 through 11 and derivatized as in the structure, above. Additionally, the present invention includes the novel fluorescent oligonucleotides having included in their sequences at least one fluorescent nucleoside analog derivatized as the phosphoramidite in the structure, above. Moreover, it is yet again another aspect of the present invention to provide fluorescent oligonucleotides made by the reactions of the aforementioned fluorescent analog 3'-O-phosphoramidites which are bound to, or have been bound by, a solid support.
3. Sources and other preparations of the fluorescent structural analogs. Formycin A is isolated as the ribonucleotide from the culture broths of *Nocardia interforma*. The antibiotic is also isolated from culture broths of *Streptomyces lavendulae* and *Streptomyces gummaensis*, and is one of numerous microbial C-ribonucleoside analogs of the N-nucleosides commonly found in RNA from all sources. The other naturally occurring C-ribonucleosides which have been isolated from microorganisms (Figure 5) include formycin B, oxoformycin B, pseudouridine, showdowmycin, pyrazomycin, and minimycin. Formycin A, formycin B, and oxoformycin B are C-nucleosides or pyrazolopyrimidine nucleosides of the class shown in Figure 6 and are structural analogs of adenosine, inosine, and hypoxanthine, respectively; a pyrazopyrimidine structural analog of guanosine obtained from natural sources has not been reported in the literature but can be chemically synthesized from the 2-chloro-formycin B or its deoxy form. A thorough review of the biosynthesis of these compounds is available in Ochi *et al*. (1974) *J. Antibiotics* xxiv.:909-916. Synthesis of the N₄ and N₆ derivatives of the C-nucleotides are described in Lewis and Townsend ([1980] *J. Am. Chem. Soc.* 102:2817). Corresponding syntheses for the isomeric aminopyrazolo[3,4d]-pyrimidines are in Wierchowski *et al*. (all others are commercially available in ribose, and several in deoxy and dideoxy forms, including the azanucleotides and deaza nucleotides, or can be synthesized *de novo*, e.g., 7-deazaadenine (Gerster *et al*. [1967] *J. Med. Chem.* 10:326)). C-nucleoside analogs of the pyrazolo-s-triazine class (e.g., pyrazolo [1,5a]-1,3,5-triazine) were prepared from amino pyrazole-C-nucleoside as originally described (Fox *et al*. [1976] *J. Heterocycl. Chem*. 13:175).

Production of the deoxy, dideoxy, and phosphorylated forms of the fluorescent ribonucleoside analogs. Chemical syntheses are available in the literature for the derivatization as 2'-deoxy forms and 3'-deoxy forms of N-nucleoside, ethenonucleosides as well as the C-nucleosides (Robins *et al*. [1973] *Can. J. Chem*. 51:1313; Jain *et al.* [1973] *J*. *Org. Chem.* 38:3719; DeClerq *et al.* [1987] *J. Med. Chem.* 30:481). Similar procedures obtain for the deoxy forms of the azanucleotides, deazanucleotides and are found in the same and additional sources (e.g., Robins *et al.* [1977] *Can. J. Chem*. 55:1251; DeClerq *et al., supra*). Protocols and procedures for synthesis of the 3'-azido, 3'amino, 2',3'-unsaturated, and 2',3'-dideoxy analogs are as reported (Lin *et al.* [1987] *J. Med. Chem.* 30:440; Serafinowski, P. [1987] *Synthesis* 10:879). Protection or derivatization of the 2'-OH with silyl or FRET moieties can be done as by Peterson and Anderson ([1989] *Silicon Compounds: Register and Review,* Petrarch Systems, Inc., pp. 60-70).

Reported herein is the novel application of a cyclic protection procedure from the ribose to the deoxyribose conversion of C-nucleosides by which only the 2'-deoxy form of the analog is produced, and by means from which high yields can be obtained without the difficult purification necessary to separate the two isomers produced using the acetoxyisobutyryl halide procedures cited above.

For enzymatic syntheses, mono- and triphosphate forms of the nucleoside analogs can be prepared by enzymatic phosphorylation with, e.g., polynucleotide kinase using established procedures, or by chemical phosphorylation. In general, the 5'-monophosphates are prepared chemically by the POCl₂ (Smith and Khorana [1958] *J. Am. Chem. Soc*. 80:1141; Yoshikawa *et al*. [1967] *Tetrahedron Lett.* 5095). The corresponding triphosphates can be chemically synthesized according to the same authors or Michelson ([1964] *Biochim. Biophys. Acta* 91:1); or Hoard and Ott ([1965] *J. Am. Chem. Soc*. 87:1785). That is, the monophosphates are treated with carbodiimide (CDI) followed with tributylammonium pyrophosphate to give the triphosphorylated form. Where it is desired to phosphorylate analogs with exposed amino groups, such substituents can be thioacetylated by treatment with ethyl trifluorothioacetate according to the procedure of Thayer *et al*. ([1974] *Biochem. J*. 139:609).

### B. Synthesis of Fluorescent Oligonucleotides

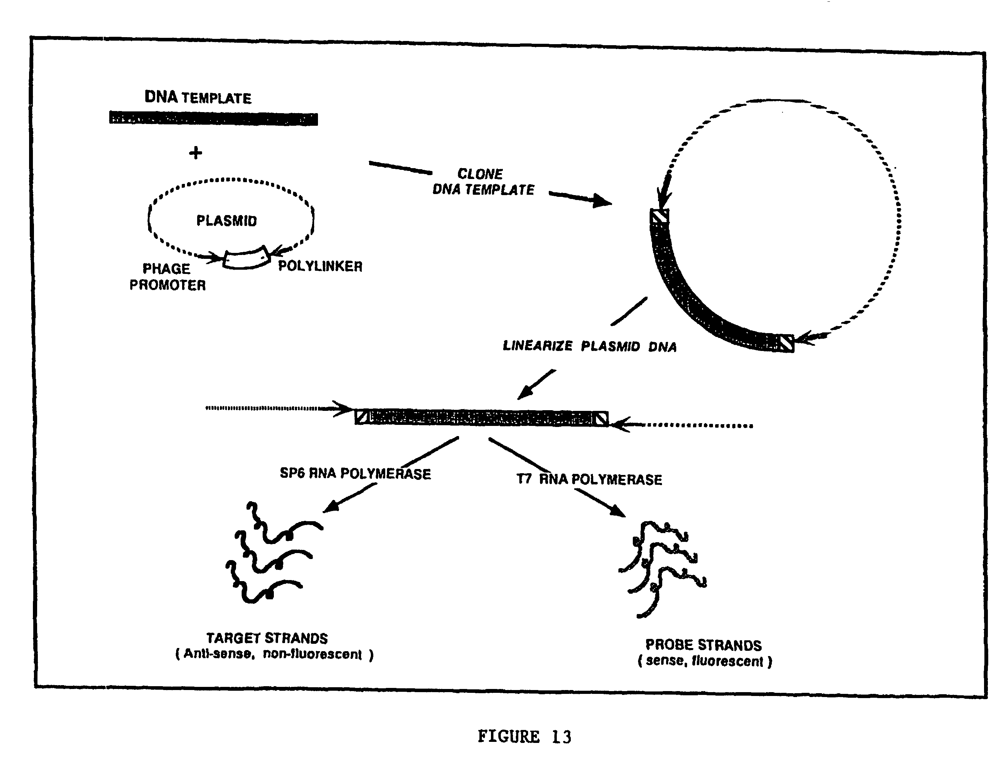

The present invention presents synthetic methods for the introduction of one or more of the fluorescent nucleoside analogs of the commonly occurring nucleotides into synthetic oligonucleotides.
1. Use of fluorescent phosphoramidites. Fluorescent phosphoramidites can be synthesized from the ribose and deoxy-ribose monomers of the fluorescent nucleoside analogs. According to the present invention, fluorescent residues are introduced into chemically synthesized oligonucleotides by first synthesizing the protected 3'-O-phosphoramidite of a nucleoside analog, e.g., 2'-deoxyformycin A; the phosphoramidite is then substituted for the corresponding standard phosphoramidite, in this case deoxy-adenosine-3'-O-phosphoramidite, and reacted with the oligonucleotide being synthesized on a solid support using standard phosphotriester chemical synthesis. The β-cyanoethyl derivatives may be selectively inserted at any desired position in a chemically synthesized oligonucleotide to produce oligomers of prescribed sequences of 60 or more bases in length and carrying any predetermined number of fluorescent bases.
   For example, non-self-hybridizing oligonucleotides were synthesized which had the perfectly alternating sequences, [AC]ₓ and [FC]ₓ, where x is the number of AC and FC dimer pairs and x had values of x=10, 15, 20, 25, 30, gave nearly identical values for both repetitive (>98%) and overall synthesis yields, and produced oligomers which differed only in that [FC]ₓ was fluorescent, whereas [AC]ₓ was not. Both olignomers hybridized specifically with complementary alternating oligomers of the sequence [TG]ₓ but not with themselves or with noncomplementary sequences such as [AG]ₓ and [TC]ₓ as indicated by (i) ethidium bromide staining in agarose gels and (ii) the melting behavior of the hybrids. Equivalent values of the melt transition temperatures in 0.075 M NaCl for the [FC]ₓ:[TG]ₓ and [AC]ₓ:[TG]ₓ hybrids varied by less than 1°C for a given value of x (length of oligonucleotide). Specifically, one aspect of the present invention involves the synthesis of 3'-O-phosphoramidites of the fluorescent nucleotides and of their fluorescent structural analogs, the use of amidites to synthesize highly fluorescent oligonucleotides having prescribed sequences and the uses of such oligonucleotides as amplification primers, fluorescent oligonucleotide "tags," and hybridization probes.
2. Use of fluorescent polyribonucleotides and polydeoxyribonucleotides. Fluorescent polyribonucleotides and polydeoxy-ribonucleotides of prescribed sequences can be synthesized enzymatically using DNA templates from a variety of sources including those prepared by chemical synthesis, cloning techniques, or obtained from genomic DNA. Representative syntheses of RNA oligonucleotides using three such DNA templates, *E. coli* RNA polymerase, the rNTPs cytidine, undine, and guanosine, together with the ribose triphosphate of either formycin A or adenosine, are illustrated in Figure 12. A representative asymmetric synthesis of an RNA probe using a template bearing directional viral promoters, the viral RNA polymerases, the rNTPS cytidine, uridine, and guanosine together with the ribose triphosphate of either formycin A or adenosine, is illustrated in Figure 13. Symmetric polydeoxyribonucleotides have been made by substituting 2'-deoxyformycin A-5'-triphosphate (FTP) for deoxyadenosine-triphosphate (dATP) in standard DNA polyerase syntheses and in DNA amplifications using thermostable DNA polymerase enzymes and the polymerase chain reaction; the corresponding asymmetric syntheses have been achieved using the same reagents and procedures but with the following modifications: (i) syntheses using such DNA polymerase as Klenow fragment or modified T7 DNA polymerase employed a template into which a primer site such as the M13 forward primer sequence was incorporated into one strand of a duplex at the beginning of the sequence that was to be used as the template, and the corresponding primer was used to initiate all syntheses; (ii) primers complementary to only one strand of a template were used in amplification as is commonly described as asymmetric PCR; or (iii) paired primers in which one of each pair of primers was coupled to a linker such as biotin were used in standard DNA amplifications such as PCR, but one strand was preferentially removed by subsequent isolation such as by use of an avidinylated column or magnetic beads. Comparable syntheses can be made by other substitutions, including, e.g., the fluorescent N-nucleosides, 2-amino purine, and 2,6-amino purine (also substituted for adenosine-5'-triphosphate) and either of the fluorescent C-nucleoside triphospates of formycin B or 5-amino-formycin B (substituted for inosine triphosphate and guanosine-triphosphate, respectively) in either their ribose and deoxyribose forms.

### C. Labeling of Fluorescent Polynucleotides

RNA and DNA can be enzymatically labeled by several methods including, but not limited to, (i) 5' DNA end-labeling using both the forward phosphorylation reaction (Richardson, C.C. [1965] *PNAS* 54:158) or the exchange kinase reaction (Van de Sande *et al*. [1973] *Biochemistry* 12:5050); (ii) mixed primer labeling by extending mixed sequence hexadeoxynucleotides annealed to restriction fragments (Feinberg, A., B. Vogelstein [1983] *Anal. Biochem*. 132:6; Feinberg, A., B. Vogelstein [1984] *Anal. Biochem.* 137:266); (iii) 3' DNA end-labeling using the enzyme, terminal deoxynucleotidyl transferase, to catalyze the repetitive addition (Okayama *et al.* [1987] *Methods Enzymol*. 154:3; Heidecker, G., J. Messing [1987] *Methods Enzymol*. 154:28) of mononucleotide units of the deoxytriphosphates, or single additions of deoxytriphosphates, of several of the fluorescent nucleoside analogs to the terminal 3'-hydroxyl of DNA initiators, including nonfluorescent probes of prescribed sequence, e.g., the *Chlamydia trachomatis* MOMP gene probe synthesized as described below; (iv) ligase labeling in which non-fluorescent "sticky-ended" or "nicked" RNA or DNA oligonucleotides are labeled by ligation with the appropriate fluorescent RNA or DNA oligomers (Pharmacia LKB [1989] *Analects* 17.2; Helfman, D.M. [1987] *Methods Enzymol*. 152:343); (v) nick translation, in which DNA polymerase is used to incorporate the triphosphates of the fluorescent analogs randomly in an existing DNA strand in a duplex (Meinkoth, J., G.M. Wahl [1987] *Methods Enzymol.* 152:91).

### D. Characterization of Fluorescent Oligonucleotides of Prescribed Sequences

Hybridization, thermal melting, agarose gel characterization and fluorescence detection studies were used to characterize oligonucleotides of prescribed sequences. In some cases, the fluorescent oligonucleotides were complementary to known sequences of target DNA from clinically important pathogens or mutations, e.g., the MOMP gene sequence from *Chlamydia trachomatis*. In these studies, the templates used for enzymatic synthesis of the fluorescent oligonucleotides were the cloned fragments also intended for use later as the target DNA in subsequent hybridization studies. Hybridization of the oligonucleotides with target DNA results in quenching of the fluorescence of the structural analogs in a fluorescent probe, which fluorescence is recovered upon denaturation of the hybrid, thereby proving that hybridization has occurred. The self-hybridization of the synthetic oligonucleotide poly(rFrU), which is discussed at length, below, is representative of the results obtained in such experiments and is summarized in Table 1.

A preferred process according to the subject invention involves four basic steps. Initially the fluorescent structural analogs are chemically or biologically synthesized and, where appropriate, further derivatized as required to synthesize a fluorescent oligonucleotide probe. Second, a DNA or RNA probe molecule complementary to a nucleic add sample of interest is constructed to have fluorescent nucleoside analogs which can be (i) distributed randomly or at specific locations throughout its length, or (ii) placed as terminal labels as described below. Third, the nucleic acid sample is then separated from unreacted monomers and can then be characterized directly, used as an extrinsic, non-specific label for tagging specific hybridization probes, or used directly as a hybridization probe. In the latter case, hybridization may take place on a solid phase to which either the target DNA/RNA or the fluorescent probe has been immobilized such as in Southern blot transfers, or "Dot-Blot" techniques, or it may occur in solution (herein, "solution hybridization"), after which probe/target hybrids are separated from unhybridized probes by simply washing or filtration. Finally, the fluorescence of the oligonucleotides hybridized to the target DNA/RNA is detected and quantified.

### E. Construction of Fluorescent Probe Molecules

In accordance with the present invention, a preselected fluorescent nucleoside analog or mixture of fluorescent analogs is substituted specifically for one or more of the non-fluorescent commonly occurring nucleosides and is then incorporated into DNA or RNA oligonucleotides to create prescribed sequences. The prescribed sequences may be chosen to be equivalent in their Watson-Crick base pairing to a nucleotide sequence constructed from normally occurring nucleotides and complementary to a given target DNA or RNA sequence; such fluorescent probes are said to be analogous to the complementary sequence of the target DNA or RNA. The fluorescent probe may be synthesized by either enzymatic or chemical synthesis for subsequent applications such as (i) hybridization probes, (ii) amplimers for direct detection of amplifiable gene sequences complementary to a given set of primers, or (iii) as non-specific "universal" labels which can be attached to specific hybridization probes by, e.g., ligation.

Fluorescent nucleoside analogs of the commonly occurring ribo-, deoxy-, or dideoxyribonucleotides can be incorporated into nucleic acid polymers using one of several otherwise conventional enzymatic and chemical techniques including, but not limited to, those described here.

### 1. Enzymatic syntheses. Enzymatic syntheses include:

(a) the use of the enzyme DNase I to introduce small "nicks" into one strand of a double stranded DNA duplex. The holoenzyme form of *E*. *coli* DNA polymerase I can then be used to extend and repair these nicks using a mixture of fluorescent nucleotide analog triphosphates, e.g., deoxyformycin-5'-triphosphate (FTP), with commonly occurring deoxynucleotide triphosphates in the reaction mixture. This method introduces a large number of fluorophores randomly throughout the DNA polymer, including both strands of the double helix. In practice, the commonly occurring nucleotide, in this case d Adenosine-5'-triphosphate (dATP), can be eliminated entirely, and the dFTP substituted in its place, without significant loss of synthetic yield, loss of hybridization specificity, or strength of duplex formation as measured by the values of the DNA melting temperature;
(b) the use of a variety of enzymes, including the Klenow fragment of DNA polymerase I and the T4 DNA polymerase, to fill in overhanging single stranded regions of DNA produced by the prior actions of restriction enzymes. This method concentrates the fluorescent analogs at the end of each DNA strand. Similarly, fluorescent DNA oligonucleotides complementary to a specific DNA template can be synthesized (i) by using DNA fragments and *E. coli* DNA polymerase, or (ii) by constructing a recombinant plasmid containing the primer site for a specific primer such as the M13 forward primer immediately 5' to the desired DNA template sequence. The DNA polymerase will synthesize a complementary DNA molecule using deoxyribonucleotides or other deoxyanalogs including, e.g., dFTP as a substitute for dATP, present in the reaction mixture;
(c) an incorporation method which also produces a terminal concentration of fluorescent analogs involves the use of the "tailing" enzyme, terminal deoxynucleotide transferase, to add a homopolymer or "tail" of fluorescent deoxy analogs to the 3' end of DNA oligomers. In practice, the yields obtained in the synthesis of homopolymers when substituting fluorescent analogs for the commonly occurring nucleosides is significantly less than the yield obtained in the synthesis of heteropolymers. Alternatively, a single fluorescent nucleoside analog may be added to the 3' OH of any oligomer using the same enzyme but the dideoxy form of a fluorescent analog or a 2'-protected fluorescent analog, including the FRET protected analogs, in exactly the same manner in which, e.g., dideoxy ATP (cordecypin), is used. A third alternative method of endlabeling hybridization probes utilizes the action of DNA ligase or RNA ligase, by which non-specific double or single stranded fluorescent oligonucleotides can be covalently coupled to either the 3' or 5' end of specific hybridization probes; the fluorescent oligonucleotides used in this fashion do not necessarily participate in the Watson-Crick base pairing which determines specificity of a probe, but may act solely as a generic or universal fluorescent "tag." With each of the foregoing methods, the DNA probes are double stranded and must be denatured to single stranded form using either heat or alkali treatment prior to their use for hybridization;
(d) an incorporation method, which can also be used as a standard method of production of fluorescent probes having a prescribed length and sequence, using standard methods of DNA amplification or replication and one of several available DNA polymerases, including but not limited to the thermostable DNA polymerases, e.g., *Taq* polymerase, modified T7 DNA polymerase, Klenow fragment, and T4 DNA polymerase, but substitutes one of the fluorescent deoxyribonucleotide analogs, e.g., 2'-deoxyformycin A-5-triphosphate or 5-aminodeoxyformycin B-5'-triphosphate for ATP and GTP, respectively, in the mix of nucleotide triphosphates. The fluorescent oligonucleotides are equivalent in yield and length to the non-fluorescent oligomer made with the commonly occurring nucleotides and hybridize to target template DNA and display the same thermal stability and capacity to stain with ethidium bromide as do the nonfluorescent controls once the hybrid duplex has formed. In such amplifications, the production of fluorescent oligonucleotides can be taken directly as evidence of the presence of a particular sequence, or the identity can be further established by (i) hybridization with a defined complementary probe, and (ii) sequencing to establish the analogous sequence; and
(e) the use of fluorescent RNA oligonucleotides complementary to a specific DNA template which can be synthesized (i) symmetrically, by using DNA fragments and, e.g., *E*. *coli* RNA polymerase as illustrated in Figure 12, or (ii) asymmetrically, as shown in Figure 13, by constructing a recombinant plasmid containing the promoter for a specific DNA dependent RNA polymerase immediately 5' to the desired DNA sequence which is used as a template, e.g., a DNA template bearing a T7 RNA polymerase promoter immediately 5' to the fragment of a cloned *Chlamydia* MOMP gene fragment which has the sequence which will be used as the target for hybridization with the probe. For most applications, asymmetric synthesis is the preferred method, and the corresponding DNA-dependent RNA polymerase will synthesize an RNA molecule using ribonucleotides, e.g., FTP as a substitute for ATP and UTP instead of TTP, which is the analogous complement to one, and only one, of the two strands of the template. The resulting single stranded probes can be used directly in a subsequent hybridization reaction without a denaturing step.
2. Chemical syntheses. The protected fluorescent deoxynucleoside analog-3'-O-phosphoramidites, typically those in which R₁₀ = dimethoxytrityl, R₁₆ = isopropyl, and R₁₅ = methyl or beta-cyanoethyl, are coupled to the 5'-OH of a growing oligonucleotide attached to a solid support using standard phosphoramidite DNA synthesis techniques (see Atkinson, T., and M. Smith [1984] In *Oligonucleotide Synthesis: A Practical Approach*, M.J. Gait, ed., IRL Press, Oxford, pp. 35-82). Solid support-bound oligonucleotide, which has already been acid washed to deprotect the 5'-OH group, is reacted with 5'-trityl protected deoxynucleoside analog-3'-O-phosphoramidite in anhydrous acetonitrile in the presence of tetrazole under argon, washing away excess reagents, and then oxidizing the phosphite product to the desired phosphate with a solution of iodine in aqueous THF, and washing with anhydrous acetonitrile. After acid washing to deprotect the new 5' terminus, the cycle can be repeated as many times as necessary to achieve the desired length and sequence; additional nucleotides which are added may be the commonly occurring nucleotides or they may be additional fluorescent nucleoside analogs. Accordingly, one or more fluorophores may be incorporated within a given probe up to and including complete substitution of, e.g., all of the A residues in a desired sequence with formycin residues. The couplings can be performed manually in a minireactor vial, utilizing a 10 minute coupling time, or on a Pharmacia LKB Gene Assembler or similar instrument utilizing the programmed synthesis protocols. The fluorescent oligonucleotide is then isolated by cleaving the DNA from the porous glass support by incubation at 55°C overnight in NH₄OH:ethanol (3:1). The fluorescent DNA containing ammonium hydroxide solution can then be quickly dried in a Speed-Vac and then separated from failure sequences of a QEAE-HPLC column using a shallow salt and pH gradient. Yields for the nucleoside analog phosphoramidites are comparable to those obtained with standard amidites based on repetitive yield calculated from trityl cation release at the deprotection step.

To provide specific illustrations of how to construct and use probe molecules containing a fluorescent nucleoside analog, following are examples which illustrate procedures, including the best mode, for practicing the invention. These examples should not be construed as limiting. All percentages are by weight and all solvent mixture proportions are by volume unless otherwise noted.

### Example 1 - Chemical Conversion of Formycin A to 2'-DeoxyFormycin A and Preparation of the 5'-Triphosphate and 3'-O-(2-cyanoethyl)-N,N,-Diisopropyl Phorphoramidite

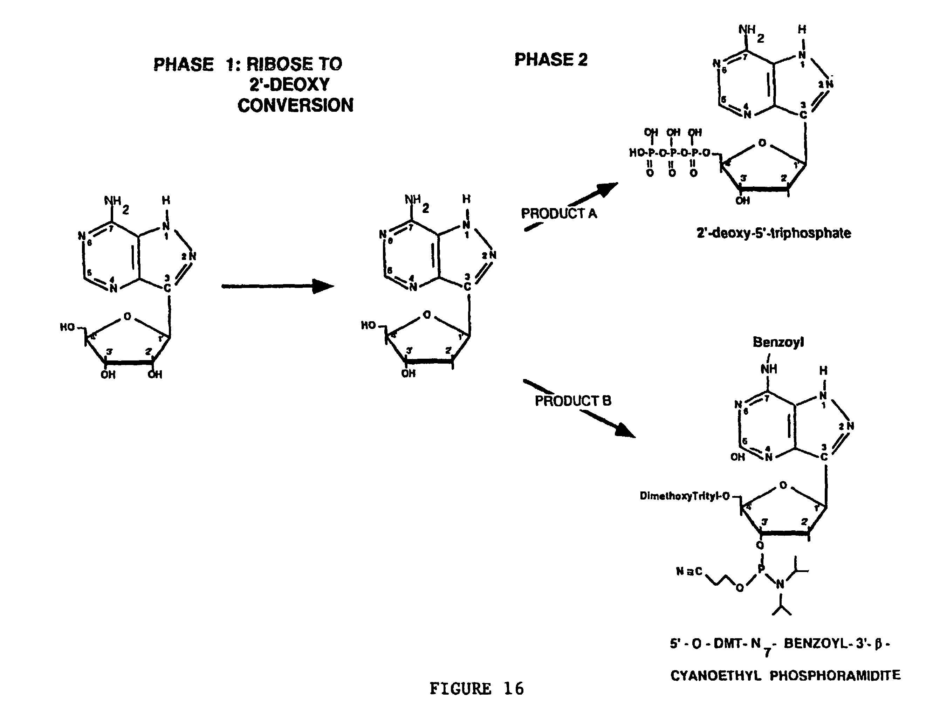

Figure 16 depicts the invention scheme used to make the 2'-deoxy-5'-triphosphate or 2'-deoxy-3'-O-phosphoramidite of formycin A. While the first phase has been previously accomplished by the reaction with α-acetoxyisobutyryl halides as described by De Clerq *et al*. ([1987] *J. Med*. *Chem*. 30:481), the procedure produces both the 3' and 2' deoxy forms which are difficult to separate and are produced in low yield. The present invention employs a 3',5'-disila protection which has previously been applied successfully in the conversion of adenosine to 2'-deoxyadenosine ([1981] *J. Am. Chem. Soc*. 103:932). The method appears to be generally applicable to the corresponding conversion of many fluorescent nucleoside analogs.

(I) 7-amino-3-[3'5'-O-(1,1,3,3-tetraisopropyl-1,3-disiloxane-dilyl)-β-D-ribofuranosyl pyrazolo [4,3d] pyrimidine. 1,3-dichloro-1,1,3,3-tetraisopropyl-1,3-disoloxane (0.9 g, 2.85 mMol) was added to a suspension of formycin A which had been exhaustively dehydrated (0.66 g, 2.5 mMol) in anhydrous pyridine and the reaction was stirred at room temperature for 24 hours. The solvent was removed under vacuum at T = 40°C and the product extracted between ethyl acetate and water. The ethyl acetate phase was washed, *in seriatim*, with (i) cold 1 N HCl, H₂O, aqueous NaHCO₃ (saturated) and aqueous NaCl (saturated) followed by evaporation to a gum. Following flash chromatography on silica gel and stepwise elution with (i) 25% methanol-chloroform, and (ii) 5% methanol-chloroform, the product, which ran as a single spot on silica TLC (R_{f} = 0.80 in 20% methanol-chloroform), was shown to be the 3',5' cyclic protected product by proton NMR and elemental analysis.

(II) 7-amino-3-[3',5'-O-(1,1,3,3-tetraisopropyl-1,3-disiloxane-dilyl)-2'-(phenoxythiocarbonyl)-β-D-ribofuranosyl] pyrazolo [4,3d] pyrimidine. 480 mg of disila protected formycin A (0.93 mMol) was dissolved with DMAP (0.9 g, 7.6 mMol) in anhydrous MeCN. Following dropwise addition of 200 mL of phenoxythiocarbonyl chloride through a dry syringe mounted in a ground glass joint, the reactants were stirred for 24 hours at room temperature, after which solvent was removed under vacuum and the product again partitioned between ethyl acetate and water. The ethyl acetate phase was washed as above, the solvent evaporated, and the residue separated on flash chromatography and eluted with chloroform-MeCN (50/50). Pooled fractions of the desired product were identified by proton NMR and elemental analysis and subjected to a second round of production, as below.

(III) 7-amino-3-(2'-deoxy-β-D-ribofuranosyl) pyrazolo [4,3d] pyrimidine (2'-deoxy formycin A). 240 mg of the product obtained from the procedure described in II, above, were added to 12.5 mg (NH₄)₂SO₄ in a gross excess of hexamethyldisilazane. The reaction mixture was refluxed at >60°C overnight. After evaporation under vacuum, the crude trisylyl derivative was redissolved in toluene and reacted with azobisisobutyronitrile and tributyl tin hydride by heating under N₂ overnight to attain complete reduction. The product was deprotected in TBAF in THF at 80°C overnight and, after evaporation, fractionated between ethyl acetate and water. The water layer was concentrated and applied to a Dowex 50W-X8 column equilibrated in water and then eluted with 15% NH₄OH. The principal product (R_{f} = 0.3 in 20% methanol-chloroform) was shown to be identical to the purified 2'-deoxy formycin A which had been prepared using the method of De Clerq *et al., supra* and by proton NMR and elemental analysis.

(IV) 7-amino-3-(2'-deoxy-β-D-ribofuranosyl) pyrazolo [4,3d] pyrimidine-5'-triphosphate (2'-deoxyformycin A-5'-triphosphate). 28 mg (0.11 mMol) of 2'-deoxyformycin A was added to a glass stoppered test tube and mixed with 02 mL of reagent grade acetone and 0.1 ml of phosphorous oxychloride. The heterogeneous reaction mixture was stored at 4°C for 24 hours, during which time the solution turned deep yellow. After cooling and addition of 3 ml cold acetone, 6 mMol of concentrated NH₄OH was added rapidly while mixing. After evaporation of the acetone, and reduction of the pH to less than 2, the mixture was refluxed for 1.5 hours, then diluted and applied directly to Dowex 1-formate, from which 2'-deoxyformycin A-MP was eluted with 0.75 M formic acid. 2'-deoxyformycin A-MP was converted to the triphosphate by the method of Yoshikawa *et al*. ([1967] *Tetrahedron Lett*. 5095).

(V) 7-amino-3-(2'-DEOXY-β-D-ribofuranosyl) pyrazolo [4,3d] pyrimidine-3'-O-phosphoramidite (2'-doexyformycin A-3'-O-phosphoramidite). 2-deoxyformycin A was treated to attain 5'-*O*- protection with DMT and benzoylation of the 7-amino group by standard procedures. To 0.3 mMol of the product and 25 mg of diisopropylammonium tetrazolide in 1.5 mL of CH₂Cl₂ was added a solution containing 0.33 mMol of *O*-cyanoethyl-N,N,N',N'-tetraisopropylphosphorodiamidite. The mixture was mixed for 4 hours and partitioned between CH₂Cl₂ and chilled in saturated NaHCO₃ solution. The CH₂Cl₂ layer was washed with saturated NaCl solution, dried (Na₂SO₄), filtered, and concentrated. Purification by filtration through a 2" plug of basic alumina in a 25 mm column, eluting with 9:1 CHCl₂/ET₃N, provided the phosphoramidite which could be dried to a foam. Identity of the product was verified by proton NMR, elemental analysis, fluorescence of the heterocycle, and use in oligonucleotide synthesis.

### Example 2 - Complete Enzymatic Substitution of FTP or 2'dFTP for ATP or dATP in RNA or DNA Probes

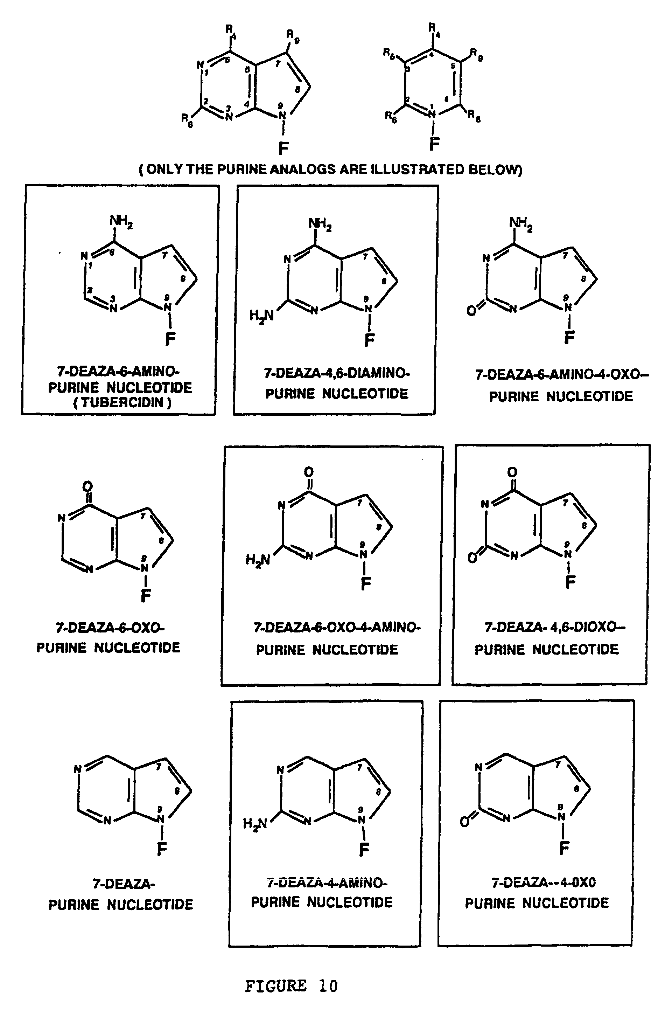

A. Symmetric synthesis of ribose oligomers. RNA oligonucleotides were synthesized from three DNA templates (Figure 10) using (i) FTP as a substitute for ATP, and (ii) a purified *E. coli* RNA polymerase as originally described by Ward *et al*. ([1969] *J. Biol. Chem*. 12:3242), except that synthesis was allowed to run for three hours at 37°C before the reaction was stopped; FTP effectively replaced ATP but not any of the other three normal nucleotides CTP, UTP, or GTP.

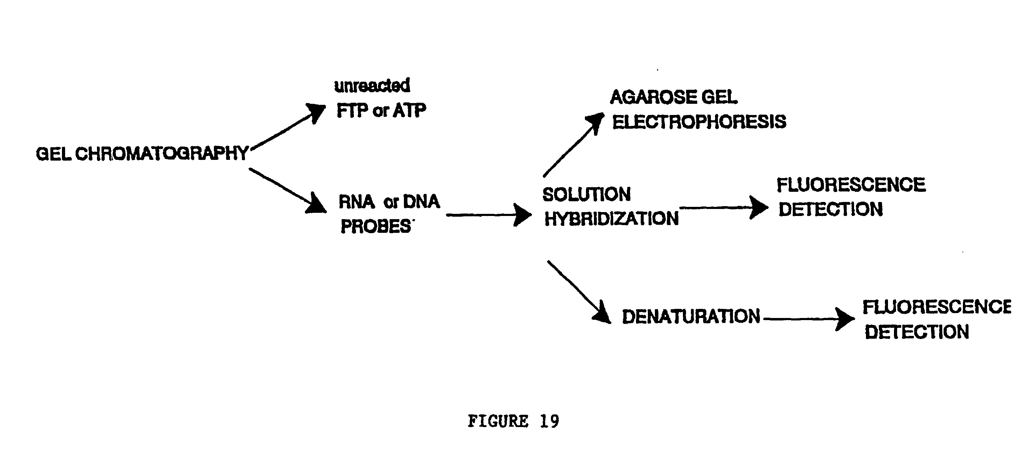

At the end of the synthesis, reaction products were separated from unreacted reagents by separation at 4°C on Sephadex G-50 in normal saline at pH 7. The scheme for separation of reaction products from unreacted agents is shown as a flow chart in Figure 19.

In the reaction, FTP is an effective substrate for RNA polymerase with both native and denatured DNA as well as with synthetic deoxynucleotide polymer templates. In samples containing CTP, UTP, GTP, RNA polymerase, one of the DNA templates, and either FTP or ATP, a high molecular weight product eluted from either sample in the void volume while the amount of monomeric NTP in the retained fraction from either sample was correspondingly reduced by >70%. No high molecular weight fraction other than the small amount of template eluted from enzyme-free controls and unreacted rNTPs were undiminished; similarly, template-free controls contained only unreacted rNTPs which co-eluted in the retained volume with standard ribonucleotide triphosphates. Similar results were obtained with a variety of DNA templates from natural and synthetic sources, including the alternating copolymers poly d(AC), poly (AG), and poly (ACGT). Moreover, comparable yields of high molecular weight oligomer were obtained from syntheses in which (i) the N-nucleoside analogs 2,6-diamino-adenosine-5'-triphosphate or 2-diamino-adenosine-5'-triphosphate were substituted for ATP in the reaction mix, or (ii) the C-nucleosides formycin B-5'-triphosphate (F_{b}TP) or -amino-formycin B-5'-triphosphate (aF_{b}TP) were substituted for GTP in the reaction mix and using poly (TG) or poly (GC) as the DNA template. No matter what the template, yields obtained by substituting several of the deaza- and aza-nucleoside analogs for ATP or GTP were dramatically lower.

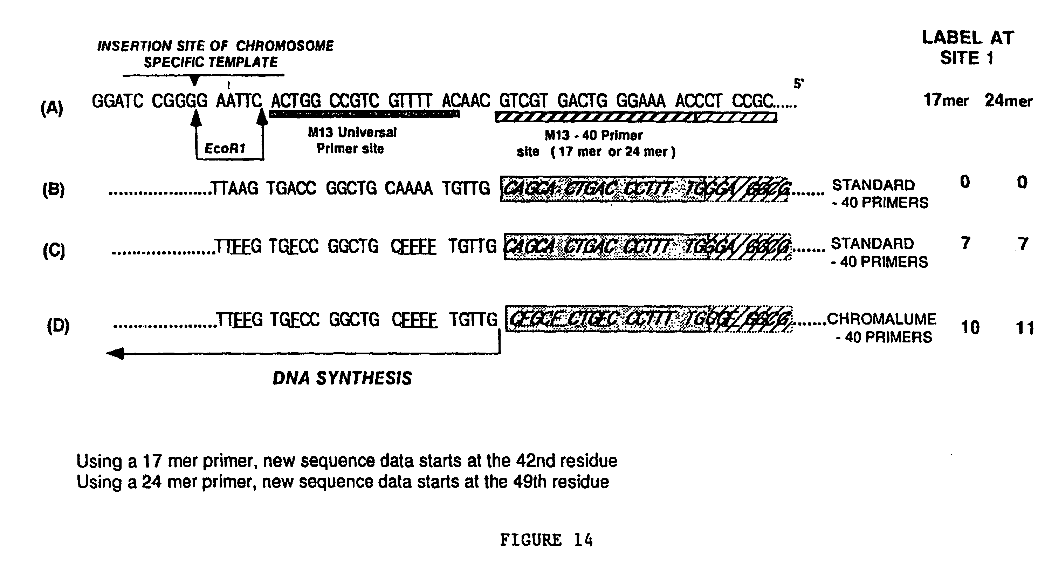

B. Asymmetric synthesis of RNA or DNA probes. *In vitro*, DNA dependent, RNA polymerase transcription systems for the synthesis of RNAs for use as substrates and hybridization probes are a fairly common tool of molecular biology. They are uniquely applied here to the development of autofluorescent probes and their production. The method developed is general and applies to any of the phage polymerase systems, including SP6, T7, and T3. In the present case, the invention employs a pair of promoters which are separately positioned on alternate strands of a duplex plasmid and at opposite ends of a polylinker as shown in Figure 13. The vectors are used to (i) attach promoters capable of effecting asymmetric synthesis through use of a viral polymerase which recognizes one of the promoters, and (ii) replicate multiple copies of a template for use in asymmetric production of a fluorescent probe or of a nonfluorescent copy of the probe target. A copy of the DNA target sequence is inserted into the polylinker in its duplex form and at a restriction site adjacent to one of the promoters. Replication of the plasmid in competent cells provides large amounts of the template for transcription. Two separate but parallel methods have been developed for the asymmetric synthesis of DNA probes. In the first case, ssDNA probes are synthesized from templates which have primer binding site attached at the 5' end of one template strand as shown in Figure 14. In such syntheses, the primer may be non-fluorescent or may be synthesized using fluorescent analog phosphoramidites as shown at the right of the Figure. A variation on this is asymmetric amplification and separation in which both strands of a template may be replicated by amplification as fluorescent oligomers, but using a pair of primers in which one, and only one, bears a transient affinity linker such as biotin which may subsequently be used to separate the denatured sense and antisense strands.

For both RNA and DNA probes, it has proven practical to establish a reference template, probe sequence, and target sequence against which all transcriptions and probe detection sensitivities are calibrated. The alpha chain of *Xenopus* translation elongation factor (Xef-1α) serves that purpose and asymmetric RNA probe synthesis is used here as representative of all RNA and DNA synthesis. The Xef-1α mRNA is a major transcription product of the *Xenopus* embryo which comprises a large percentage of the non-mitochondrial mRNA transcripts that appear immediately after the midblastula transition. The gene for the Xef-1α was isolated and *Eco*RI linker sites added at the ends of the clone during construction of the cDNA library. The 1705 nucleotide fragment was inserted into a pSP72 plasmid bearing a T7 promoter on one strand and an SP6 promoter on the complement. Following plasmid replication and template linearization, transcription with T7 RNA polymerase, the rNTPs cytidine, uridine, and guanosine, together with the ribose triphosphate of either formycin A or adenosine, produced 1749-base-long oligomers containing 489 F or A residues, respectively. Transcripts less than full length were never observed and, in each case, the analogous and control oligomers were produced in comparable quantities and were generally indistinguishable in physical behavior save that the analogous sequence was permanently fluorescent.

There are two unique features of this novel manufacturing system. (1) Synthesis of the antisense strand, e.g., using SP6 and the commonly occurring nonfluorescent rNTPs provides standardized target sequences in high yield. In the corresponding asymmetric synthesis of DNA probes, distinct primer sites on complementary template strands can be used to achieve the same objective. (2) A mixture of plasmids containing several different plasmids can be used to create a "cocktail" of linearized templates from which the corresponding "cocktail" of probes (see Example 7, below), which can bind to multiple sites on a genomic sequence, can be concurrently transcribed.

### Example 3 - The Fluorescence of Nucleoside Analog RNA Probes and Proof of Their Hybridization in Solution

The effective utilization of FTP in the poly d(AT) directed synthesis in Example 1 produced a polymer approximately 300-500 bases in length which, when hydrolyzed and/or sequenced, proved to be a perfectly alternating replicate of the DNA template, but with the sequence: poly (FU). As predicted from this sequence, the product could be annealed to like chains by a single thermal cycle, thereby creating the putative product poly (FU):poly (FU); unlike the comparably treated poly (FC), which showed no evidence of self-hybridization as expected, the annealed hybrids of poly (FU):poly (FU) stained with ethidium bromide in agarose gels and gave a sharp thermal transition in both absorbance and fluorescence, proving that the probes could hybridize both effectively and specifically. The absorbance and emission spectra of the purified poly (FU), poly (FC), poly (FG), poly (UF_{b}), poly (CaF_{b}), and poly (FCGU) differ from those of purified poly (AU), poly (AC), poly (AG), poly (TG), and poly (ACGT) controls in four respects: (i) the far UV absorbance maximum is shifted slightly for the analog-containing products, to 265 nm as compared to 260 nm for the controls; (ii) there is a significant, highly structured absorbance (3 peaks at room temperature) between 290 nm and 320 nm with negligible absorbance at 340 nm; (iii) an excitation maximum appears at 303 nm; and (iv) there is a broad emission band extending into the visible wavelengths with a peak at 405 nm (Stokes shift = 102 nm). It is an important property that the fluorescence is fully quenched in, e.g., the poly (FU):poly (FU) hybrid, and cannot be detected until the strands are denatured by raising the pH of the solution to values >pH 10. Once denatured, the fluorescence of the oligomer is fully integratable, with relative fluorescence intensity >40% of peak intensity over the range 360 nm to 460 nm.

**Table 1.**

| **Properties of hybrid formation by poly (AU) and poly (FU)** | | | | | | |
|---|---|---|---|---|---|---|
| RNA:RNA HYBRID | DENATURED HYBRID | | | INTACT HYBRID | | |
| | WAVELENGTH MAXIMA | | | LENGTH (BASE PAIRS) | ETHIDIUM BROMIDE STAINING | MELT TEMP |
| | ABSORBANCE | EXCITATION | EMISSION | | | |
| r[AU]:r[AU] | 260 nm | ---- | ―-- | 150-300 | yes | 32°C |
| r[FU]:r[FU] | 266 nm | 303 nm | 405 nm | 150-300 | yes | 33°C |

### Example 4 - Hybridization of Fluorescent Probes to Target RNAs and Target DNAs; Uses of Linkers to Allow Solid Phase Detection

The synthetic template poly (TG) was used to produce the complementary RNA probes poly (AC) and poly (FC), neither of which is self complementary and in which hybrids could not be annealed or detected; of the two only the poly (FC) was fluorescent. In a parallel experiment, a poly (AC) template was amplified using the biotinylated synthetic 22-mer primers, ^{5°}BIOTIN-(TG)₁₁^{3'}, together with standard polymerase chain reaction (PCR) methods to produce the DNA amplimers having the sequence, ^{5°}BIOTIN-poly (TG)^{3'}, then separated from the unreacted primers by gel sizing and/or QEAE ion exchange chromatography, after which the polymers were radioactively labeled using ³²P-ATP and the enzyme polynucleotide kinase. When mixed separately, but in equimolar amounts, with the biotinylated amplimers, ^{5°}BIOTIN-poly (TG)^{3'}, both of the RNA probes, poly (AC) and poly (FC), formed hybrids which could be characterized by (i) ethidium bromide staining, and (ii) melting behavior, as expected, the fluorescence of the poly (FC) probe was quenched by hybridization. The hybrids could then be adsorbed via the ^{5'}BIOTIN moiety to avidinylated beads, washed to remove unhybridized poly (FC), and equal aliquots assayed for radioactivity and fluorescence. Prior to denaturation of the washed sample, detectable fluorescence in the solution was negligible; when denatured in high pH buffer, the amount of poly (FC) which had been hybridized, when estimated from the fluorescence of standardized dilutions of the probe, was within 1% of the amount of the target DNA, ^{5°}BIOTIN-poly (TG)^{3'}, as measured by the amount of radioactive label in the sample as compared to standardized dilutions.

### Example 5 - Hybridization of Fluorescent Probes Synthesized from Nucleoside Analog-3'-O-Phosphoramidites to Target DNAs

In a validation of the use of the phosphoramidites of the fluorescent nucleoside analogs, n-mers which varied in length in multiples of 5 bases from 25-mers to 60-mers, and having the sequence (AC)ₓ or (FC)ₓ, where x = 12.5, 15, 17.5, 20, 22.5, 25, 27.5, or 30, were synthesized in parallel using either dAdenosine-3'-O-phosphoramidite or dF-3'-O-phosphoramidite together with dC-3'-O-phosphoramidite in a Pharmacia LKB Gene Assembler. After cleavage from the solid phase and purification of QEAE-Sepharose, the fluorescent oligomers (FC)ₓ of defined length could be hybridized to the radiolabeled amplimers of poly (TG), from Examples 2 and 3, above, as assessed by DNA melting behavior, ethidium bromide staining, and the reappearance if quenched fluorescence following denaturation of the hybrid.

### Example 6 - Assay for Chlamydia trachomatis Using an FTP Substituted RNA Probe

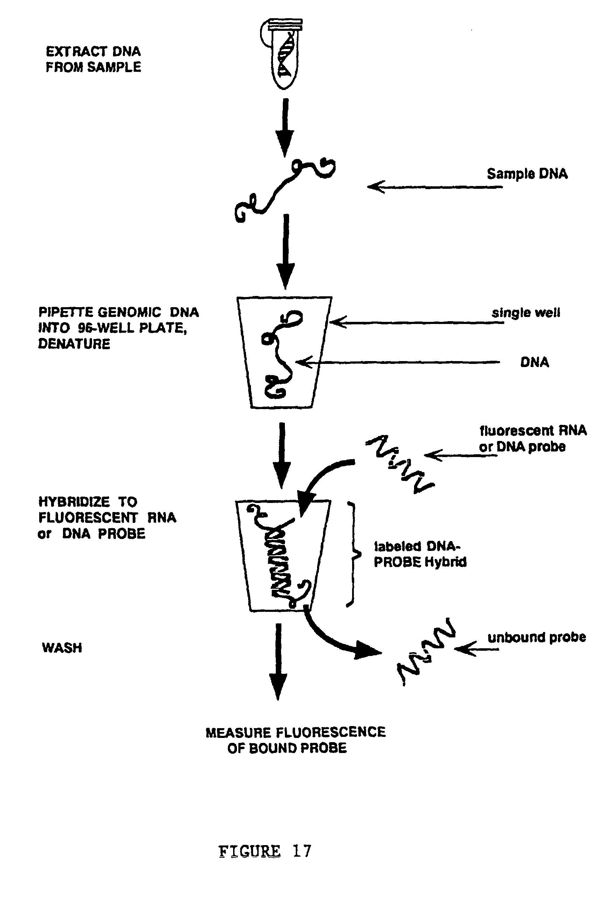

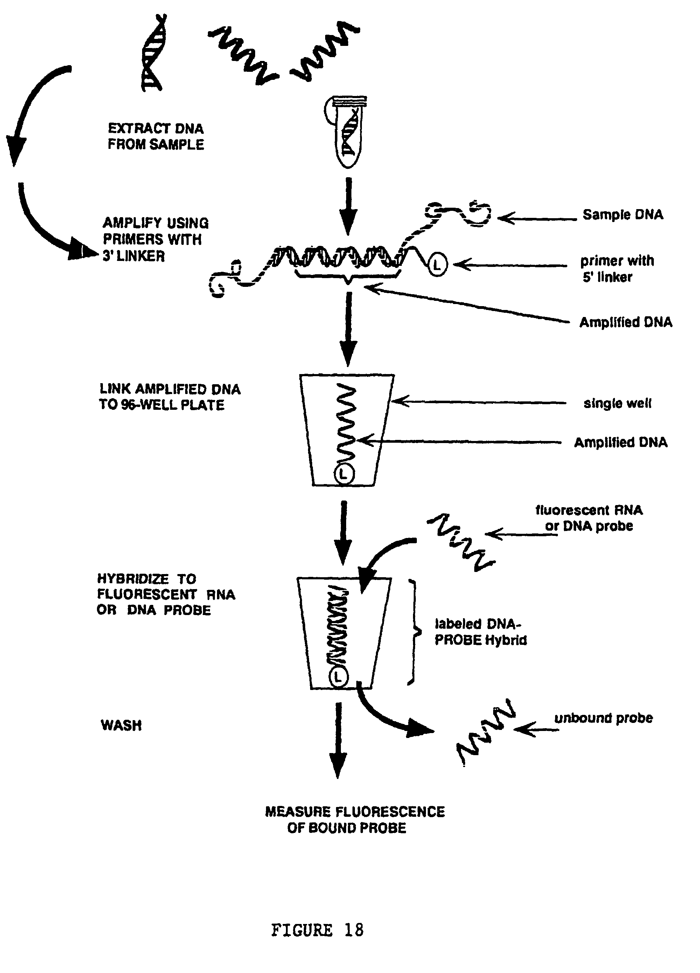

*Chlamydia trachomatis* is an obligatory intracellular pathogen which, in its active infectious stages, contains from 3x10³ to 4x10³ copies of ribosomal RNA (rRNA) and one copy of genomic DNA/bacterium. A primer pair, one of which contained a 5'-biotinylated T7 promoter which was 5' to the hybridizing primer sequence, was used to amplify a 150 base pair DNA segment of the MOMP gene from a stock strain of *C. trachomatis* L2. Approximately 500 ng of the DNA fragment, which contained the T7 RNA polymerase promoter at the 5' end, was transcribed with T7 RNA polymerase in the presence of rCTP, rUTP, rGTP, and with either rFTP or rATP (+ control). The reaction was stopped by heat inactivating the enzyme for 3 minutes at 100°C. Unincorporated rNTPs were separated from the labeled RNA by gel sizing chromatography on a Sephadex G-25 column, after which the probe concentration was estimated from its absorbance at 260 nm. Using a simple dual monochromator fluorescence spectrophotometer, as little as 5 x 10⁻¹⁴ moles of the RNA probe could be detected over background when 20 nm slits were used for both excitation and emission monochromators. A photon counting fluorimeter designed for sensitivity (see Example 9, below) is capable of detecting between 5 x 10⁻¹⁶ and 5 x 10⁻¹⁷ moles of the same probe, equivalent to the amount of ribosomal RNA expected from between 5000 to 50,000 of the bacteria. Two hundred microliters of either (i) *C. trachomatis* genomic DNA, or (ii) the amplified target DNA were mixed with 200 µL of a 1/200 dilution of the probe in hybridization buffer (0.15 M NaCl, 0.02 M sodium citrate, 0.02 M HEPES, 0.004 M EDTA, pH 7.4) and the mixture boiled for 3 minutes, after which they were allowed to cool slowly to room temperature over one hour. An aliquot of the genomic DNA sample was eluted into an ultrafiltration microtube or 96-well filter plate (pore size = 0.1 µm) as illustrated in Figure 17, washed 5 times with 0.15 M NaCl, 0.02 M sodium citrate, pH 7.4, after which the sample was divided in two, one half denatured in high pH buffer, and both aliquots scanned to measure fluorescence background and the fluorescence of hybridized probe, respectively. Target DNA amplimers were treated similarly except that the 5'-biotinylated primer end of the target DNA segments were first adsorbed to avidinylated magnetic beads (2.8 µm diameter) so that the sample could be washed without loss of material (Figure 18). With either treatment, fluorescence of the probe may be detected at dilutions of the sample which contain less than 1 x 10⁻¹⁶ moles of target DNA, which is roughly equivalent to the sensitivity required to detect less than 10,000 bacteria if a single similarly sized probe were used to detect rRNA from infectious *Chlamydia*. The probe used here is about 150 bases in length, contains approximately 38 formycin residues per probe, and binds only to a single target site on each copy of the ribosomal RNA. It is an important feature of this invention that increasing the number of fluorophores in a probe, or probe "cocktail," also increases the sensitivity of detection. With 13 times as many formycin residues per probe as the 150 base MOMP gene probe, 1 x 10⁻¹⁸ moles of the Xef-1α probe can be detected in a dual monochromator fluorescence spectrophotometer whereas less than 1 x 10⁻²⁰ moles are detected using the photon counting technology described in Example 9.

### Example 7 - Detection of Multiple Target Sites

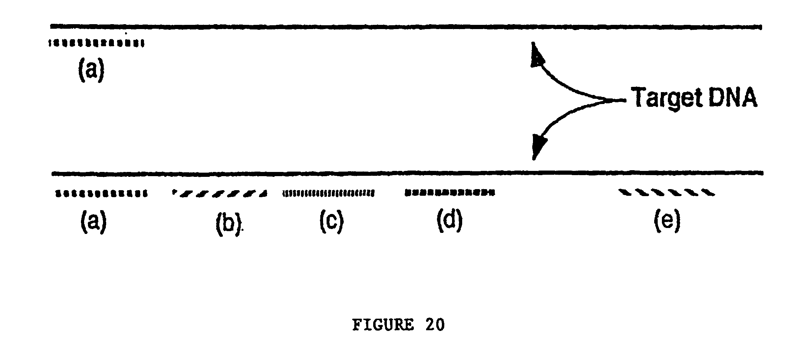

An important aspect of the asymmetric syntheses to both diagnostic and therapeutic, e.g., antisense, applications of nucleic add probes is the capacity for concurrent synthesis of probe "cocktails" which may comprise probes which differ in length or differ in the locations or numbers of the target sites on RNA or genomic DNA to which they will bind. Utilization of probe cocktails to three different types of diagnostic targets illustrate the broad importance of this feature.
A. Single target nucleic acids present in multiple copies. In some species of pathogen, multiple copies of rRNA are present in each organism, e.g., each bacterium of *Chlamydia trachomatis* contains approximately 2 x 10⁴ rRNA molecules per organism. Since the rRNA of *Chlamydia* is typically between 3000 and 5000 nucleotides in length, sensitivity in a diagnostic assay may be increased significantly by use of a probe cocktail specific for target sequences on rRNA and made of as many as 5 to 10 different probe sequences, each of which can bind to discrete segments of the target rRNA or target DNA as indicated with probes (a) to (e) in the lower half of the diagram shown in Figure 20 in which (a), (b), (c), (d), and (e) are analogous complementary probes specific for different target sequences of a single DNA strand.
   There are two disadvantages in using rRNA sequences as diagnostic targets: (i) rRNA sequences are highly conserved, hence only short variable sequences are useful for the detection and identification of infectious pathogens. One consequence of this to diagnostic sensitivity is that only limited numbers of 'reporter' labels can be used on each probe, thereby limiting sensitivity; and (ii) only a few pathogens carry rRNA in high copy numbers, and many, such as the DNA viruses, carry no rRNA at all, hence the number of diagnostics which can employ this strategy is limited.
B. Multiple different target sequences on a single strand of DNA The genomes of all organisms are significantly larger than rRNA and typically carry more numerous and larger unique segments which can serve as target sequences for nucleic acid probe hybridization. For example, the complete genome of *Chlamydia trachomatis* has been isolated and consists of a relative small double stranded DNA with a molecular weight of >660 x 10⁶ or slightly more than 1 x 10⁶ base pairs. Each bacterium also contains a 4.4 x 10⁶ dalton plasmid containing >7 kbases. Unlike the rRNA of this species, the plasmid is unique to *Chlamydia* in its entirety―no cross-hybridization can be detected with the DNA from, e.g., *Neisseria gonorrhea*―indeed, no cross-hybridization occurs between the different restriction fragments of the plasmid itself. Even when no other portion of the *Chlamydia* genomic DNA is chosen for use as hybridization targets, a cocktail specific for the multiple restriction fragments of the *Chlamydia* plasmid alone is equivalent in length to more than 4 Xef-1α probes and can be detected at levels equivalent to between 100 and 1000 bacteria.
C. Multiple copies of a single target sequence on a single strand of DNA. It has only recently been discovered that flanking sequences on each side of several genes contain moderate to long stretches of tandem repeats. Ribosomal gene repeats are of particular interest in the kinds of DNA based diagnosis described in this invention. Like the ribosomal genes, they are present in high copy numbers, which improves sensitivity of detection but, in addition, the spacer regions between genes are normally highly variable from species to species, since they are not subject to selective pressures. Multiple copies of the same unique sequence on a single DNA strand represents a special case in which the hybridization targets are a cocktail of loci on each genome; that is, a single probe sequence can probe multiple target sites of the same sequence and on the same DNA strand. They are ideally suited as species and genus specific probe targets.
   A representative example of such probes and targets was created for the different species of the protozoan parasite *Eimeria*, which causes coccidiosis in a variety of domestic animals. Genomic DNA from *E. tenella* was digested with several different restriction enzymes, and the fragments ligated into appropriately cut asymmetric plasmid vectors and were used to transform *Escherichia coli*. Colonies were screened for repeat sequences by hybridization with *Eimeria tenella* genomic DNA that had been labeled with ³⁵S by random priming. Strongly hybridizing clones were picked and subjected to differential screening with labeled genomic DNA from *E. mitis, E. maxima, E. acervulina*, and *E. tenella*, as well as DNA from the closely related genera *Plasmodium*, *Trypanosoma*, and *Sarcocystis*. The majority of clones gave signals of equal intensity with DNA from the other genera. Some clones, however, were recognized specifically by the *Eimeria* and one clone was recognized only by *E. tenella*.
   The entire sequence of the insert in the latter clone contains 334 base pairs. Physical characterization of the restriction fragments indicates that the sequence is present in tandemly repeated units of approximately 738 base pairs and that a minimum of 30 genes are tandemly linked and all appear to be on one chromosome. Asymmetric probes synthesized using the tandem repeat as a template contain 179 formycin A residues per template sequence.
   Even when no other portion of the *Eimeria* genomic DNA is chosen for use as a hybridization target, a single sequence probe specific for only the multiple copies of the tandem repeat on the *Eimeria* genome is equivalent in length to more than 11 Xef-1α probes. Since the infectious particles for *Eimeria* are oocysts, each of which contains 8 genomes, such cocktail of targets makes it possible to detect less than 10 oocysts. The import of tandem repeat targets extends well beyond sensitivity, however, or simply the detection of this single genus, since tandem repeat sequences appear in a genomic DNA of a wide variety of species and genera, and are distinct for those species, thereby providing a broad basis for the design of diagnostic assays for a wide variety of pathogens, including those for which no rRNA targets exist.

### Example 8 - The Use of Non-Specific and Non-Hybridizing Fluorescent Oligomers as Universal Fluorescent "Tags" by Ligation or Chemical Linkage

Simple modification of the template to produce a "sticky end" at the 3', 5', or both 3' and 5' termini, e.g., to ^{5'}ACGT-polyd(AT), polyd(AT)-TGCA^{3'}, or ^{5'}ACGT-polyd(AT)-TGCA^{3'}, respectively, enabled synthesis of RNA probes with all of the above properties, but which could also be ligated, either (i) to like strands to produce longer fluorescent probes, or (ii) to other hybridization sequences specific for a prescribed target DNA. The latter is a particularly useful way in which to produce a universal label for any cloned DNA fragment, and allows a given probe to be identified by two non-hybridizing but highly fluorescent sequences at its termini, without the need to denature the hybrid for detection as was seen with the simple poly (FU) probe, above. Equivalent non-hybridizing universal probes can be readily made by chemical synthesis using, e.g., the etheno analog phosphoramidites, e.g., 1,N₆-ethenoAdenosine-3'-O-phosphoramidite (eA), to synthesize non-specific tags which can subsequently be linked to any hybridization probe. In general, the 3' or 5' termini of such universal probes can also be prepared for chemical, rather than enzymatic attachment to other oligomers or solid phases, through the addition of, e.g., 5'-amino hexyl, 5'-sulfhydryl hexyl, 3'-aminohexyl amino, N-hydroxysuccinimide esters, and other such linkers.

### Example 9 - Quantitation of Luminescent Probe Using Time Resolved Fluorometry

A novel method for detecting fluorescent nucleoside analogs, fluorescent oligonucleotides or analogous sequences, of the amount of bound fluorescent oligonucleotide probe has been developed based on the use of photon counting to measure the amount of a fluorophore in a sample and is described herein below. The method differs from time resolved spectroscopy in that the method integrates all fluorescence emission from a fluorophore or nucleic acid probe, independent of the wavelength of the emission and is both a novel combination of time and spectral integration and a novel application of photon counting to the identification, detection, and quantitation of nucleic acid target sequences to diagnostic assays and therapeutic treatments.

The fundamental experimental parameter used in any measurement of luminescence is the intensity of the luminescence, *l*, the units of which are moles of photons per second per liter. Because the fluorescent nucleoside analogs used here are, for all practical purposes, permanently fluorescent and do not photobleach within the lifetime of a typical measurement, the luminescence of fluorescence, measured in moles of photons emitted per second per mole of fluorophore, can be used as an index of the amount of fluorophore, and hence probe, in a sample. The preferred instrumentation for such measurements, developed at Chromagen, comprises (i) a 150 watt Hg/Xe CW cylindrical lamp capable of high intensity excitation over the range 290 nm ≤ λ ≤ 320 nm, (ii) an ultrahigh sensitivity photomultiplier in which the photodynode is coated to allow a response only over the range of emission 360 nm ≤ λ ≤ 550 nm, (iii) a cylindrical cuvette with quartz excitation windows but glass walls which can serve as the emission filter. The cuvette is mounted so that the entire sample can be collected at the face of the photomultiplier tube, and (iv) 5 computer-driven photon counting clocks, connected *in seriatim*, and each capable of discriminating between photons at a frequency of 10⁹ per second.

In experiments with the monomeric formycin A and full-length Xef-1α probe containing 489 formycin residues under conditions of room temperature and pH = 10, we have found that (i) the luminescence of serial dilutions of the monomer and the probe are linearly related to the concentration, and (ii) the luminescence of the probe is equivalent to the same number of free monomers. In a typical assay using permanent fluorophores such as those shown in Figures 17 and 18, the amount of target present in a sample is determined by denaturing hybrids after unbound probe has been washed away and measuring the amount of probe which was bound. The fluorescence equivalence of residues in an analogous probe sequence to the emission of the same number of monomers, under alkaline conditions used here, indicates that there is negligible self-quenching in the oligomer and demonstrates that the luminescence of the probe can be used directly to quantitate the amount of probe bound by target RNA or DNA, thereby providing a broad basis for the design of diagnostic detectors for a wide variety of nucleic acid assays and diagnostics. It is an important consequence of the invention, that sensitivity and signal-to-noise ratios are a function of the number of the photons counted and the number of time periods over which counting is done.

### Example 10 - Attachment of 5' and 3' Linkers for Immobilization of the Oligonucleotides and Hybrids or for Attachment of Fluorescent Oligomers as "Labels"

The chemistries and procedures of the invention can be used to create and characterize any probe synthesized using fluorescent nucleoside analogs, whether the synthesis is enzymatic or chemical, for both fluorescence and hybridization specificity. Such probes can be used not only in the solution hybridization formats described here, but also in the more frequently used laboratory procedures such as "dot-blot" detection, electrophoresis in agarose or polyacrylamide gels, Southern blotting, and hybridization on filters and membranes, as well as separation of the hybrids by HPLC or capillary electrophoresis methods. Although linkers are not essential to the solution hybridization, any appropriate affinity linker such as biotin/avidin or homo- or heterobifunctional linker can be used to capture the probe or hybrid for purposes of concentration, isolation, or detection, as illustrated for the PCR amplified DNA fragments of Figure 13. The present invention includes linker derivatized fluorescent nucleotides, as well as oligonucleotides, linker derivatized primers for use in amplification and subsequent detection with fluorescent oligonucleotide probes, oligonucleotide probes, plasmids, and therapeutics made or otherwise "tagged" therefrom, and/or their uses and applications such as are described herein. Such derivatizations include, but are not limited to, transaminations to purine or pyrimidine nucleosides and/or their fluorescent structural analogs, amino-thiol, azido-, aldehyde, hydroxysuccinimide, 5' aminoalkyl-3'-O-phosphoramidite, 5'-thioalkyl-3'-O-phosphoramidite, 3'-aminohexyl amino, amino silanes, and aminosilyl derivatives and other such linkers and groups reactive with linkers or in condensation reactions such as Schiff base condensations of 3' or 5' oxidized *cis*-diols, as are familiar to one skilled in the art. To illustrate this a specific case is offered:
(i) a set of non-fluorescent amplification primers for the MOMP gene sequence was chemically synthesized; at the end of synthesis an additional cycle was used to add 5'-aminohezyl-3'-O-phosphoramidite to the 5' terminus of the completed primer with the addition chemically synthesized, using standard phosphotriester chemistry.
(ii) Following cleavage from the solid phase support in strong ethanolic base, the terminal amino group of each strand was reacted with NHS-biotin ester to provide the 5' biotinylated primers.
(iii) The primers were used for standard amplification, after which the amplimers were captured on avidinylated 96-well filter plates and washed to remove unreacted materials and contaminants.
(iv) The captured amplimers were hybridized with fluorescent analog labeled oligonucleotide probes as described above and the amount of target sequence in the amplimers quantified.

Included in the present invention are such attachments of fluorescent oligonucleotides to other fluorescent or non-fluorescent oligonucleotides to immobilizing beads, filters, or activated plastic plates and done through enzymatic attachment such as ligation, or chemical attachment through such linkers as are described herein.

### Example 11 - Uses of Fluorescence Resonance Energy Transfer (FRET) to Broaden or Enhance the Uses of Fluorescent Nucleoside Analogs and Probes

Oligonucleotides can be synthesized or derivatized as described herein which have two or more spectrally distinct, detectable labels, either by using two or more nucleoside analogs with discrete fluorescence emission characteristics, or by use of a covalently attached FRET acceptor, such as is described hereinabove. FRET acceptors can also be used to enhance or broaden the sensitivity of the detection for the fluorescent probes, if they are simply available in solution to act as acceptors of the probe emission. For example, the excitation spectra of such dyes as the coumarins, e.g., 7-amino-4-methylcoumarin-3-acetate, 7-methyl-umbelliferone, the naphthalene and anthracene dyes, etc., overlap the emission spectrum of oligomers constructed from the fluorescent nucleoside analogs, e.g., poly (FU), but not the oligomers' excitation spectrum. Such dyes as 7-amino-4-methylcoumarin-3-acetate may thus be used either (i) as a covalently attached FRET acceptor, e.g., by reacting the N-hydroxysuccinimide ester with prescribed amino groups on the oligomer, or (ii) by simply adding the dye to a solution of the probe to act as a FRET indicator of probe fluorescence. In addition to the obvious advantages of providing a second fluorescent label to the hybridization probe, this methodology allows amplification of the probe signal through more efficient capture of the emitted light, reduction of background light due to light scattering from excitation sources, and detection at longer visible wavelengths.

It should be understood that the examples and embodiments described herein are for illustrative purposes only and that various modifications or changes in light thereof will be suggested to persons skilled in the art and are to be included within the spirit and purview of this application and the scope of the appended claims.

## Claims

1. A method for the detection of a target nucleotide in a sample, which comprises contacting the sample, under hybridisation conditions, with an oligonucleotide probe that specifically hybridises with the target, and detecting hybridisation by observing fluorescence or a change in fluorescence; **characterised in that** the probe includes a fluorescent nucleoside instead of any of the six commonly occurring non-fluorescent nucleosides.

2. A method according to claim 1, wherein the fluorescent nucleoside is at the 3' or 5' end of the probe.

3. A method according to claim 2, wherein the probe is between 5 and 10,000 bases in length.

4. A method according to any of claims 1 to 3, wherein the probe further comprises a 5' to 3'linker group having affinity for or chemically binding to a solid support selected from glass, agarose, acrylamide, nylon and nitrocellulose.

5. A method according to any of claims 1 to 4, wherein hybridisation is detected by the quenching of fluorescence.

6. A method according to any of claims 1 to 5, wherein the fluorescent nucleoside has either following structure 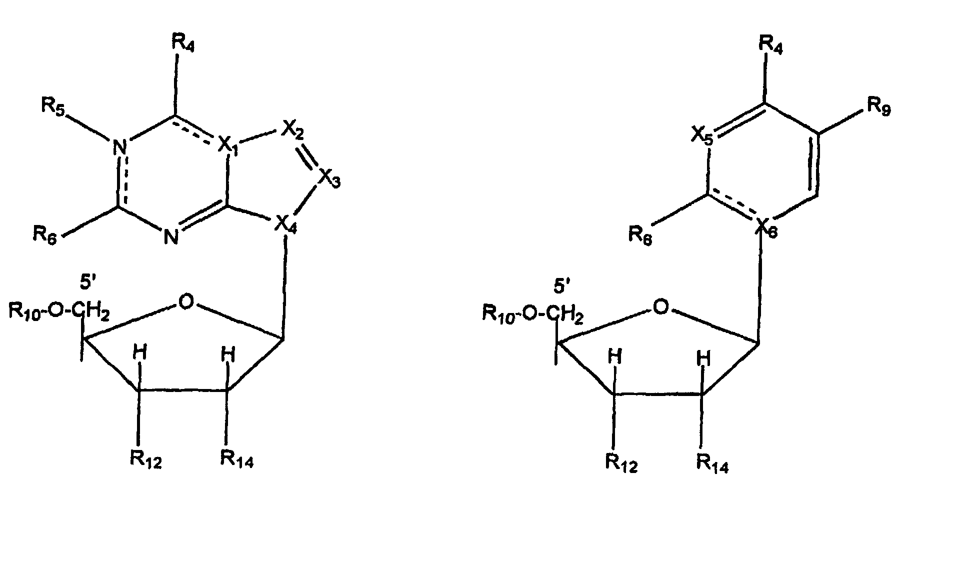 wherein --- X₁ and --- X₆ are each =C, =Si or -N;
X₂ is CR₉, SiR₉ or N;
X₃ is CR₈, SiR₈ or N;
X₄ and X₅ are each CH, SiH or N;
provided that at least one of X₁, X₂, X₃, X₄, X₅ and X₆ is N;
R₄ is NH₂, SH or OH;
--- N-R₅ is =N, -NH or -N-CH=CH- linked to R₄;
R₆ is H, NH₂, SH, OH or =O;
R₈ and R₉ are independently H, Br, F, I, alkyl or aryl;
R₁₀ is H, an acid-sensitive, base-stable blocking group, or a phosphorus derivative;
R₁₂ is H, OH, amino, azido, thiol, an unsaturated group, or a phosphorus derivative; and
R₁₄ is H, OH or OR₃ wherein R₃ is a protecting group or fluorophore;
with the overall proviso that at least one of R₁₂ and R₁₄ is H.

7. A method according to claim 6, wherein R₁₀ is selected from H, monophosphate, diphosphate, triphosphate, β,γ-methylene-2'-triphosphate, 5'-O-phosphoramidite, phosphodiester, methylphosphonate, phosphorothioate, phosphoramidite and phosphotriester.

8. A method according to claim 6, wherein
R₁₂ is OP(OR₁₅)-N(R₁₆)₂;
R₁₄ is H;
R₁₅ is methyl, β-cyanoethyl, p-nitrophenyl, o-chloronitrophenyl or p-chlorophenyl; and
R₁₆ is lower alkyl, preferably methyl or isopropyl, or heterocyclic, such as morpholino, pyrrolidino or 2,2,6,6-trimethylpyrrolidino.

9. A method according to claim 6, which comprises
(A) combining single stranded nucleic acid from the sample with the fluorescent probe, 5 to about 10,000 bases in length,
and wherein
(i) the sequence of the fluorescent oligonucleotide probe is analogous to the complementary sequence of the portion of the target DNA or RNA to which it is meant to hybridize;
(ii) the fluorescent oligonucleotide probe is capable of Watson-Crick base pairing such that each fluorescent nucleoside analog forms base pairs only with the complement of the commonly occurring nucleotide for which it has been substituted; and
(iii) the derivation of single-stranded nucleic acid with said fluorescent oligonucleotide probe is carried out under conditions that stable duplexes or hybrids form (a) only between the fluorescent oligonucleotide probes and that portion or sequence of the target DNA or RNA present in the sample to which the complementary sequence to the target DNA or RNA would bind; but (b) not significantly between fluorescent oligonucleotide probe and non-target DNA or RNA in the fragments thereof; and
(B) determining whether stable duplex was formed in step (A) by:
(i) (a) separating the unhybridized fluorescent oligonucleotide probe from hybridized fluorescent probe:target nucleic acid duplex formed in step (A); or (b) binding the duplexed fluorescent oligonucleotide probe, if needed, to a solid phase to facilitate washing and/or concentration;
(ii) denaturing the isolated hybrids; and
(iii) determining whether a detectable signal is present by the treatment of (A)(iii)(a) as an indicator of the presence of the biological entity or genetic mutation in the sample.

10. A method according to claim 9, wherein the presence of a target nucleic acid sequence in a sample is tested by amplification using primers which have been modified at their 5' termini to enable specific chemical or affinity linkage, adsorption, or binding to a solid support, said support comprising polystyrene beads, 96 well plates, agarose, polyacrylamide, nylon, or nitrocellulose.

11. A method according to claim 6, which comprises:
(A) incorporating a ribonucleotide or deoxyribonucleotide, modified by the incorporation or attachment thereto of fluorescent nucleotide analogues, into a polynucleotide complementary or analogous to the complement to said polynucleotide; and
(B) hybridising said complementary fluorescent or analogous complementary fluorescent oligonucleotide to said target polynucleotide; and detecting the presence of said nucleotides by the fluorescence of the probe.

12. A method according to claim 11, wherein the hybridising step or detecting step is carried out on a solid phase.

13. A kit for the determination of the presence of target nucleotide sequence in the nucleic acid of a biological sample, which comprises:
(A) a primer polynucleotide comprising a primer sequence substantially complementary to a target nucleotide sequence in a biological sample, wherein said target nucleotide sequence comprises an oligonucleotide segment complementary to the 3' terminal of said primer to form a template for primer-dependent nucleic acid polymerase;
(B) a plurality of nucleotide triphosphates wherein at least one of said triphosphates is a fluorescent nucleoside analogue;
(C) a primer-dependent DNA polymerase which extends the primers in a 5' to 3' direction when the 3' terminus of the primer is base-paired and hybridised to a template DNA sequence; and
(D) a fluorescent oligonucleotide probe comprising an analogous complementary sequence to said target oligonculeotide, which can strongly and specifically hybridise to said target sequence for detection, identification, location or quantification of said target nucleotide sequence.

## Patentansprüche

1. Verfahren zum Nachweis eines Ziel-Nukleotids in einer Probe, welches umfasst, die Probe unter Hybridisierungsbedingungen mit einer Oligonukleotid-Sonde, die mit dem Ziel spezifisch hybridisiert, in Kontakt zu bringen und eine Hybridisierung nachzuweisen, indem eine Fluoreszenz oder eine Änderung der Fluoreszenz beobachtet wird, **dadurch gekennzeichnet, dass** die Sonde ein fluoreszierendes Nukleosid anstelle von einem beliebigen der sechs üblicherweise vorkommenden nicht-fluoreszierenden Nukleoside umfasst.

2. Verfahren nach Anspruch 1, wobei das fluoreszierende Nukleosid sich am 3'- oder 5'-Ende der Sonde befindet.

3. Verfahren nach Anspruch 2, wobei die Sonde 5 bis 10000 Basen lang ist.

4. Verfahren nach einem der Ansprüche 1 bis 3, wobei die Sonde ferner eine 5'→ 3'-Linkergruppe umfasst, die eine Affinität für einen festen Träger, der aus Glas, Agarose, Acrylamid, Nylon und Nitrocellulose ausgewählt wird, aufweist oder chemisch an einen solchen bindet.

5. Verfahren nach einem der Ansprüche 1 bis 4, wobei eine Hybridisierung durch das Quenchen von Fluoreszenz nachgewiesen wird.

6. Verfahren nach einem der Ansprüche 1 bis 5, wobei das fluoreszierende Nukleosid eine der folgenden Strukturen aufweist 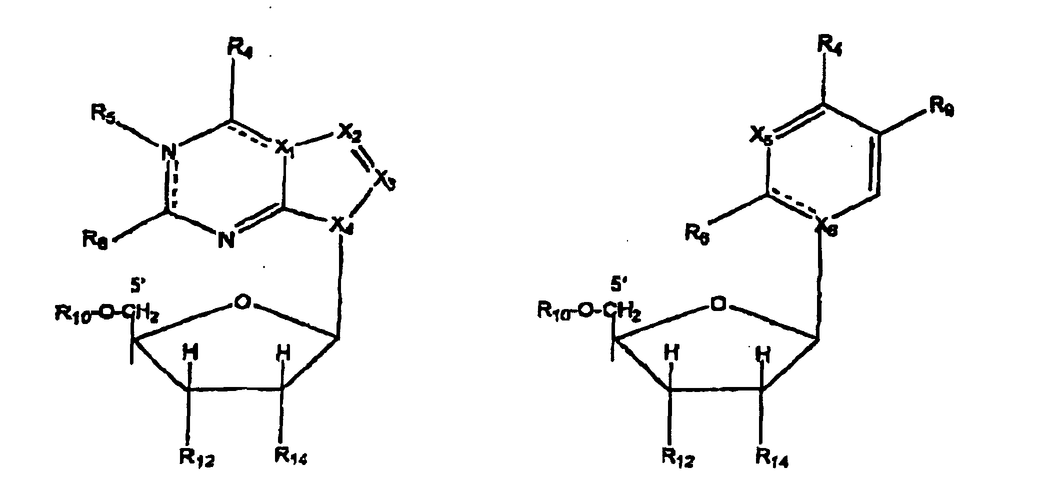 worin ---X₁ und ---X₆ jeweils =C, =Si oder -N sind;
X₂ CR₉, SiR₉ oder N ist;
X₃ CR₈, SiR₈ oder N ist;
X₄ und X₅ jeweils CH, SiH oder N sind;
mit der Maßgabe, dass wenigstens eines von X₁, X₂, X₃, X₄, X₅
und X₆ N ist;
R₄ NH₂, SH oder OH ist;
---N-R₅ =N, -NH oder -N-CH=CH-, das an R₄ gebunden ist, ist;
R₆ H, NH₂, SH, OH oder =O ist;
R₈ und R₉ unabhängig voneinander H, Br, F, I, Alkyl oder Aryl ist;
R₁₀ H, eine säureempfindliche, basensrabile Blockierungsgruppe
oder ein Phosphorderivat ist;
R₁₂ H, OH, Amino, Azido, Thiol, eine ungesättigte Gruppe oder ein Phosphorderivat ist; und
R₁₄ H, OH oder OR₃ ist, wobei R₃ eine Schutzgruppe oder ein Fluorophor ist;
mit der insgesamten Maßgabe, dass wenigstens eines von R₁₂ und R₁₄ H ist.

7. Verfahren nach Anspruch 6, wobei R₁₀ aus H, Monophosphat, Diphosphat, Triphosphat, β,γ-Methylen-2'-triphosphat, 5'-O-Phosphoramidit, Phosphodiester, Methylphosphonat, Phosphorthioat, Phosphoramidit und Phosphotriester ausgewählt wird.

8. Verfahren nach Anspruch 6, wobei
R₁₂ OP(OR₁₅)-N(R₁₆)₂ ist;
R₁₄ H ist;
R₁₅ Methyl, β-Cyanethyl, p-Nitrophenyl, o-Chlornitrophenyl oder p-Chlorphenyl ist; und
R₁₆ Niederalkyl, vorzugsweise Methyl oder Isopropyl, oder heterocyclisch, wie Morpholino, Pyrrolidino oder 2,2,6,6-Trimethylpyrrolidino, ist.

9. Verfahren nach Anspruch 6, welches umfasst,
(A) einzelsträngige Nukleinsäure aus der Probe zusammen zu bringen mit der fluoreszierenden Sonde von 5 bis ungefähr 10000 Basen Länge, und wobei
(i) die Sequenz der fluoreszierenden Oligonukleotid-Sonde analog zu der komplementären Sequenz des Abschnitts der Ziel-DNA oder -RNA, mit dem sie hybridisieren soll, ist;
(ii) die fluoreszierende Oligonukleotid-Sonde zu einer Watson-Crick-Basenpaarung in der Lage ist, derart, dass jedes fluoreszierende Nukleosidanalog Basenpaare nur mit dem komplementären Molekül des üblicherweise vorkommenden Nukleotids, das durch dieses ausgetauscht worden ist, bildet; und
(iii) die Ableitung von einzelsträngiger Nukleinsäure mit der fluoreszierenden Oligonukleotid-Sonde unter Bedingungen ausgeführt wird, dass stabile Doppelstränge oder Hybride sich (a) nur zwischen den fluoreszierenden Oligonukleotid-Sonden und jenem Abschnitt oder jener Sequenz der in der Probe vorhandenen Ziel-DNA oder -RNA, an den oder die die zu der Ziel-DNA oder -RNA komplementäre Sequenz binden würde, bilden; aber sich (b) nicht signifikant zwischen fluoreszierender Oligonukleotid-Sonde und Nicht-Ziel-DNA oder -RNA in den Fragmenten davon bilden; und
(B) zu bestimmen, ob in Schritt (A) ein stabiler Doppelstrang gebildet worden ist, durch:
(i) (a) Trennen der nicht-hybridisierten fluoreszierenden Oligonukleotid-Sonde von hybridisierten fluoreszierende Sonde:Ziel-Nukleinsäure-Doppelsträngen, die in Schritt (A) gebildet worden sind; oder (b) Binden der in einen Doppelstrang eingebauten fluoreszierenden Oligonukleotid-Sonde, sofern erforderlich, an eine feste Phase, um ein Waschen und/oder eine Aufkonzentrierung zu vereinfachen;
(ii) Denaturieren der isolierten Hybride; und
(iii) Bestimmen, ob ein detektierbares Signal vorhanden ist, durch die Behandlung von (A) (iii) (a) als einen Indikator für das Vorhandensein der biologischen Einheit oder einer genetischen Mutation in der Probe.

10. Verfahren nach Anspruch 9, wobei das Vorhandensein einer Ziel-Nukleinsäuresequenz in einer Probe getestet wird durch Amplifizierung unter Verwendung von Primern, die an ihren 5'-Enden modifiziert worden sind, um eine spezifische chemische oder Affinitäts-Verknüpfung, Adsorption oder Bindung an einen festen Träger zu ermöglichen, wobei der Träger Polystyrol-Körnchen (-Beads), Platten mit 96 Vertiefungen, Agarose, Polyacrylamid, Nylon oder Nitrocellulose umfasst.

11. Verfahren nach Anspruch 6, welches umfasst:
(A) Einbauen eines Ribonukleotids oder Desoxyribonukleotids, das durch Einbauen oder Anheftung daran von fluoreszierenden Nukleotidanalogen modifiziert worden ist, in ein Polynukleotid, das komplementär oder zu dem zu dem Polynukleotid komplementären Strang analog ist; und
(B) Hybridisieren des komplementären fluoreszierenden oder analogen komplementären fluoreszierenden Oligonukleotids an das Ziel-Polynukleotid; und Detektieren des Vorhandenseins der Nukleotide durch die Fluoreszenz der Sonde.

12. Verfahren nach Anspruch 11, wobei der Hybridisierungsschritt oder Detektionsschritt an einer festen Phase ausgeführt wird.

13. Kit zur Bestimmung des Vorhandenseins einer Ziel-Nukleotidsequenz in der Nukleinsäure einer biologischen Probe, welcher umfasst:
(A) ein Primer-Polynukleotid, umfassend eine Primersequenz, die im wesentlichen komplementär zu einer Ziel-Nukleotidsequenz in einer biologischen Probe ist, wobei die Ziel-Nukleotidsequenz ein Oligonukleotidsegment umfasst, welches zu dem 3'-terminalen Abschnitt des Primers komplementär ist, um eine Matrize für eine Primer-abhängige Nukleinsäurepolymerase zu bilden;
(B) eine Mehrzahl von Nukleotidtriphosphaten, wobei wenigstens eines der Triphosphate ein fluoreszierendes Nukleosidanalog ist;
(C) eine Primer-abhängige DNA-Polymerase, die die Primer in einer 5'→ 3'-Richtung verlängert, wenn das 3'-Ende des Primers Basenpaarungen zu einer Matrizen-DNA-Sequenz ausbildet und an diese hybridisiert; und
(D) eine fluoreszierende Oligonukleotid-Sonde, umfassend eine analoge komplementäre Sequenz zu dem Ziel-Oligonukleotid, welche stark und spezifisch mit der Zielsequenz für einen Nachweis, eine Identifizierung, Lokalisierung und Quantifizierung der Ziel-Nukleotidsequenz hybridisieren kann.

## Revendications

1. Méthode de détection d'un nucléotide cible dans un échantillon, qui consiste à mettre en contact l'échantillon, dans des conditions d'hybridation, avec une sonde oligonucléotidique qui s'hybride spécifiquement avec la cible, et à détecter l'hybridation en observant une fluorescence ou un changement de fluorescence, **caractérisée en ce que** la sonde comprend un nucléoside fluorescent au lieu de l'un quelconque des six nucléosides courants, non fluorescents.

2. Méthode selon la revendication 1, dans laquelle le nucléoside fluorescent est à l'extrémité 3' ou 5' de la sonde.

3. Méthode selon la revendication 2, dans laquelle la sonde a une longueur de 5 à 10000 bases.

4. Méthode selon l'une quelconque des revendications 1 à 3, dans laquelle la sonde comporte en outre un groupe de liaison 5' → 3' ayant une affinité pour, ou se liant chimiquement à, un support solide sélectionné parmi le verre, l'agarose, l'acrylamide, le nylon et la nitrocellulose.

5. Méthode selon l'une quelconque des revendications 1 à 4, dans laquelle l'hybridation est détectée par l'extinction de la fluorescence.

6. Méthode selon l'une quelconque des revendications 1 à 5, dans laquelle le nucléoside fluorescent présente l'une ou l'autre des structures suivantes: 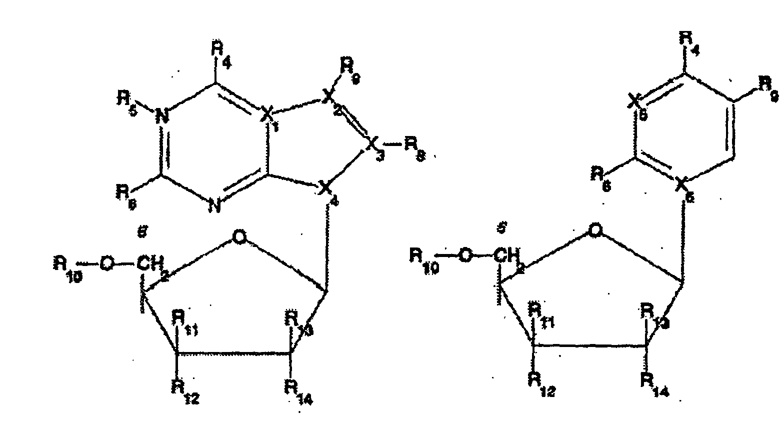 où =X₁ et =X₆ représentent chacun =C, =Si ou -N;
X₂ représente CR₉, SiR₉ ou N;
X₃ représente CR₈, SiR₈ ou N;
X₄ et X₅ représentent chacun CH, SiH ou N;
à condition qu'au moins l'un parmi X₁, X₂, X₃, X₄, X₅ et X₆ représente N;
R₄ représente NH₂, SH ou OH;
=N-R₅ représente =N, -NH ou -N-CH=CH- lié à R₄;
R₆ représente H, NH₂, SH, OH ou =O;
R₈ et R₉ représentent indépendamment H, Br, F, I, un alkyle ou un aryle;
R₁₀ représente H, un groupe bloquant sensible aux acides, résistant aux bases, ou un dérivé phosphoré;
R₁₂ représente H, OH, un amino, un azido, un thiol, un groupe insaturé ou un dérivé phosphoré;
R₁₄ représente H, OH ou OR₃, où R₃ représente un groupe protecteur ou fluorophore;
avec la condition générale qu'au moins l'un parmi R₁₂ et R₁₄ représente H.

7. Méthode selon la revendication 6, dans laquelle R₁₀ représente H, un monophosphate, un diphosphate, un triphosphate, un β,γ-méthylène-2'-triphosphate, un 5'-O-phosphora-midite, un phosphodiester, méthylphosphonate, un phosphorothioate, un phosphoramidite et un phosphotriester.

8. Méthode selon la revendication 6, dans laquelle
R₁₂ représente OP(OR₁₅)-N(R₁₆)₂;
R₁₄ représente H;
R₁₅ représente un méthyle, un β-cyanoéthyle, un p-nitrophényle, un o-chloronitrophényle ou un p-chlorophényle; et
R₁₆ représente un groupe alkyle inférieur, de préférence méthyle ou isopropyle, ou un groupe hétérocyclique, p. ex. morpholino, pyrrolidino ou 2,2, 6, 6-triméthylpyrrolidino.

9. Méthode selon la revendication 6, qui consiste
(A) à combiner un acide nucléique monocaténaire de l'échantillon avec la sonde fluorescente, d'une longueur de 5 à environ 10000 bases, et dans laquelle
(i) la séquence de la sonde oligonucléotidique fluorescente est analogue à la séquence complémentaire de la partie de l'ADN ou de l'ARN cible à laquelle elle doit s'hybrider;
(ii) la sonde oligonucléotidique fluorescente est capable d'un appariement Watson-Crick tel que chaque analogue nucléosidique fluorescent forme des paires de bases uniquement avec le complément du nucléotide courant qu'il a remplacé; et
(iii) la dérivation de l'acide nucléique monocaténaire par ladite sonde oligonucléotidique fluorescente est effectuée dans des conditions telles que des hybrides ou des duplex stables se forment (a) uniquement entre les sondes oligonucléotidiques fluorescentes et la portion ou séquence de l'ADN ou de l'ARN cible présente dans l'échantillon à laquelle la séquence complémentaire de l'ADN ou de l'ARN cible se lierait; mais (b) pas de manière significative entre la sonde oligonucléotidique fluorescente et de l'ADN ou l'ARN non-cible dans les fragments; et
(B) à déterminer si un duplex stable s'est formé à l'étape (A), en:
(i) (a) séparant la sonde oligonucléotidique fluorescente non hybridée du duplex sonde/acide nucléique cible hybridé fluorescent formé à l'étape (A); ou (b) fixant la sonde oligonucléotidique fluorescente duplexée, si nécessaire, à une phase solide pour faciliter le lavage et/ou la concentration;
(ii) dénaturant les hybrides isolés; et
(iii) déterminant si un signal détectable est présent par le traitement de (A) (iii) (a) comme indicateur de la présence de l'entité biologique ou d'une mutation génétique dans l'échantillon.

10. Méthode selon la revendication 9, dans laquelle la présence d'une séquence d'acide nucléique cible dans un échantillon est testée par amplification au moyen d'amorces qui ont été modifiées à leurs extrémités 5' pour permettre une liaison spécifique chimique ou par affinité, une adsorption ou une liaison à un support solide, ledit support comprenant des billes de polystyrène, des plaques à 96 puits, de l'agarose, du polyacrylamide, du nylon ou de la nitrocellulose.

11. Méthode selon la revendication 6, comportant:
(A) l'incorporation d'un ribonucléotide ou d'un désoxyribonucléotide, modifié par l'incorporation ou l'attachement à lui d'analogues nucléotidiques fluorescents, dans un polynucléotide complémentaire ou analogue au complément dudit polynucléotide; et
(B) l'hybridation dudit oligonucléotide fluorescent complémentaire ou analogue au complément fluorescent, audit polynucléotide cible; et la détection de la présence desdits nucléotides par la fluorescence de la sonde.

12. Méthode selon la revendication 11, dans laquelle l'étape d'hybridation ou l'étape de détection est conduite sur une phase solide.

13. Trousse pour la détermination de la présence d'une séquence nucléotidique cible dans l'acide nucléique d'un échantillon biologique, qui comprend:
(A) une amorce polynucléotidique comportant une séquence amorce essentiellement complémentaire d'une séquence nucléotidique cible dans un échantillon biologique, dans laquelle ladite séquence nucléotidique cible comporte un segment oligonucléotidique complémentaire de l'extrémité 3' de ladite amorce pour former une matrice pour l'acide nucléique polymérase dépendante d'amorce;
(B) une pluralité de nucléotides triphosphates où au moins l'un desdits triphosphates est un analogue nucléosidique fluorescent;
(C) une ADN-polymérase dépendante d'amorce qui allonge les amorces dans le sens 5' → 3' lorsque l'extrémité 3' de l'amorce montre un appariement de bases et est hybridée à une séquence ADN matrice; et
(D) une sonde oligonucléotidique fluorescente comportant une séquence complémentaire analogue dudit oligonucléotide cible, qui peut s'hybrider fortement et spécifiquement à ladite séquence cible pour la détection, l'identification, la localisation ou la quantification de ladite séquence nucléotidique cible.
